# EUROPEAN PATENT APPLICATION

(11) **EP 3 431 593 A1**
(43) Date of publication of application: **23.01.2019**
(21) Application number: 17766464.6
(22) Date of filing: 07.03.2017
(51) Int. Cl.: C12N 15/09, C12N 5/071, C12N 5/10

(54) **METHOD FOR DIRECTLY PRODUCING CARDIAC PRECURSOR CELL OR MYOCARDIAL CELL FROM FIBROBLAST**

(30) Priority: 15.03.2016 JP 2016051407
(71) Applicant: Keio University, Tokyo 108-8345 (JP); National Institute of Advanced Industrial Science and Technology, Tokyo 100-8921 (JP)
(72) Inventor: IEDA, Masaki, Tokyo 160-8582 (JP); SADAHIRO, Taketaro, Tokyo 160-8582 (JP); ISOMI, Mari, Tokyo 160-8582 (JP); GOSHIMA, Naoki, Tokyo 135-0064 (JP)
(74) Representative: Vossius & Partner Patentanwälte Rechtsanwälte mbB
(86) International application number: PCT/JP2017/009052
(87) International publication number: WO 2017/159463

(57) **Abstract**

Provided is a method of inducing cardiac progenitor cells or cardiomyocytes from fibroblasts. The present invention provides a method for producing cardiac progenitor cells, comprising introducing one cardiac reprogramming factor into fibroblasts, or a method for producing cardiomyocytes, comprising introducing three cardiac reprogramming factors into fibroblasts.

SEQUENCE LISTING

<110>  Keio University
       National Institute of Advanced Industrial Science and
       Technology

<120>  Direct production of cardiac progenitor cells and cardiac
       myocytes from fibroblasts

<130>  G1521WO

<140>  JP2016-051407
<141>  2016-03-15

<160>  12

<170>  PatentIn version 3.5

<210>  1
<211>  1830
<212>  DNA
<213>  Homo sapiens

<400>  1
agctgtcgga ctcaccgggg gccctaagca gcgagacctg aggccagacg gaactacaac      60

atgtaccatc cacgagaatt gtacccgtcc ctgggggccg gctaccgcct ggggcccgcc     120

caacctgggg ccgactccag cttcccaccc gccctagcgg agggctaccg ctaccccgaa     180

ctggacaccc ctaaactgga ttgcttcctc tccgggatgg aggctgctcc ccgcaccctg     240

gccgcgcacc cacctctgcc ccttctgccc cctgccatgg gcactgagcc ggccccatca     300

gctccagagg ccctccattc cctcccgggg gtcagcctga gcctggagaa ccgggagcta     360

tggaaggagt tcagctctgt gggaacagaa atgatcatca ccaaagctgg gaggcgcatg     420

ttccctgcct gccgagtgtc agtcactggc ctggaccccg aggcccgcta cttgtttctt     480

ctggatgtga ttccggtgga tggggctcgc taccgctggc agggccggcg ctgggagccc     540

agcggcaagg cagagccccg cctgcctgac cgtgtctaca ttcaccccga ctctcctgcc     600

actggtgcac attggatgcg gcagcctgtg tctttccatc gtgtcaagct caccaacagc     660

acgctggacc cccacggcca cctgatcctg cactccatgc acaagtacca accccgcata     720

cacctagttc gggcagccca gctctgcagc cagcactggg ggggcatggc ctccttccgc     780

ttccccgaga ccacattcat ctccgtgaca gcctaccaga acccacagat cacacaactg     840

aagattgcag ccaatccctt tgccaaaggc ttccgggaga acggcagaaa ctgtaagagg     900

gagcgagacg cccgtgtgaa gaggaaactg cggggcccag agccagcagc cacagaggcc     960

tatgggagcg gagacacacc aggtggtccc tgcgactcca ccctgggtgg agacattcgt    1020

gaatcagatc cagaacaggc cccagccccc ggggaagcca ccgctgcccc ggcacctctg    1080

tgtggtggcc ccagtgctga ggcctacctc ctgcaccctg cggctttcca tggggccccc    1140

agtcaccttc ccaccaggag ccccagcttc ccggaggctc cagactccgg gcgctcagcc    1200

ccctactcgg ctgcatttct ggagctgccg cacgggtcag ggggctccgg gtacccagcg 1260

gctccaccgg cggtaccctt tgccccgcac tttctccaag gggccccttt ccctctacca 1320

tacaccgcgc ctgggggcta tctggatgtg ggctccaaac ccatgtactg aaccactgct 1380

gggcccctct gcctccatca caaacctccc gctcccttcc cccagccctg gagccccctc 1440

acctccaccg gccccatccc ccacaccaaa tggccgcctt gggcctgccc acccaccccc 1500

tcaacttcac accttgattt cactcccacc cccctctggc ttcaaagcct aaccaaggct 1560

gctgggagtc cagctggggc cggcttcccc ttcccggctc tcacctccgt gtgaatggaa 1620

ggggctctgc ctggccaaat ggggccacag accagcatcc ccacttacca ggcctcccca 1680

tgggctctgg aggggctcaa tccccacccc cccacacaca ctggtgcagg ccacaccagt 1740

ctgttgttct gggaccagag tatttttgtt aataaaactc aaaagccatc catgctcagg 1800

aaaaaaaaaa aaaaaaaaaa aaaaaaaaaa 1830

<210> 2
<211> 436
<212> PRT
<213> Homo sapiens

<400> 2

<210>  3
<211>  1731
<212>  DNA
<213>  Mus musculus

<400>  3
cacaaggcca gaagaaacta caacatgtac catccacgag agttgtaccc ctccctgggg      60

actggctacc gtctgggaca cccccagcct ggggcagact ccaccttccc acctgccctg     120

acagagggtt accgctaccc tgatttggat acttctaaac tggattgctt cctctctggg     180

atcgaggcag ctccccacac tctggctgca gccgctcctt tgccccttct cccatctgct     240

ctgggccccg agacagcacc gccaccccca gaggcccttc actcgcttcc tggggtcagc     300

ctgagcttgg agaaccagga actgtggaag gaattcagcg ctgtggggac agagatgatc     360

atcaccaagg ctggcaggcg catgttccct gcttgccgag tatcagtcac tggcctggac     420

ccagaggccc gctacttgtt tcttctggat gtggttccag tggatggggc ccgataccgc     480

tggcagggcc agcactggga gccaagtggc aaggctgaac cccgcctacc cgaccgtgtc     540

tacattcacc ctgactctcc tgccactggt gcccactgga tgcggcagcc cgtatccttc     600

catcgtgtta agctcaccaa cagcacactg gacccccatg gccacctgat cttgcactcg     660

atgcacaagt accagcctcg catccacctg gtgagagcca cccaactatg cagccaacac     720

tgggggggtg tggcctcctt ccgatttcct gagaccacat tcatctctgt gacagcctac     780

cagaacccta ggatcacaca gctgaagatc gcagccaatc cctttgccaa aggtttccga     840

gaaaatggca gaaactgtaa gagggagcgg gatgcccgtg tgaagaggaa acttcggggc     900

ccagagccag tggccacaga ggcctgtggg agtggggata caccaggggg tccctgtgac     960

tccaccctgg gtggggacat tcgggactca gatccagagc aggccccaac cccccaggaa    1020

gctgcttctg cctcagctcc tccatgtggg ggccccagtg ctgaggccta ccttctacac    1080

cctgccgctt ttcatggcgc ccccagtcac ctaccagcca ggacccccag cttcgctgag    1140

gctccagacc ctgggcgccc agccccctac tcagctgcat ttctggacct acagcctgga    1200

ccagggggct ctgcctatca ggcagctcca tctgtaccat cctttgcccc acacttcatc 1260

caagggggtc ccttccctct accgtaccca ggacctggag gttatctgga catgggatcc 1320

aagccaatgt actgagcctc ggtgtagtaa ccctatgcca tcttcccttg atcctccagc 1380

tcccttcccc cagcctggta gcatccgcat tgaagtggta tccccctccc ccccaccaaa 1440

tggctggctt gggcctctct tccacccttt agttcacacc ttgatttcac tccaccccct 1500

ctggcttcaa agctcaggca aggcagctct gagtccagct ggggcctgct tcccctcagc 1560

tctcacctta gtgcgaatgg agggagcctc tgcctggcca aatggggctc ccagcccagt 1620

acccccacct cctgggggcc ttgttactag tgcaagccat gccaatttgt tctcaggatc 1680

agagtatttt tgttaataaa actccaaaga cattaaaaaa aaaaaaaaaa a 1731

<210> 4
<211> 436
<212> PRT
<213> Mus musculus

<400> 4

Met Tyr His Pro Arg Glu Leu Tyr Pro Ser Leu Gly Thr Gly Tyr Arg
1               5                   10                  15

Leu Gly His Pro Gln Pro Gly Ala Asp Ser Thr Phe Pro Pro Ala Leu
            20                  25                  30

Thr Glu Gly Tyr Arg Tyr Pro Asp Leu Asp Thr Ser Lys Leu Asp Cys
        35                  40                  45

Phe Leu Ser Gly Ile Glu Ala Ala Pro His Thr Leu Ala Ala Ala Ala
    50                  55                  60

Pro Leu Pro Leu Leu Pro Ser Ala Leu Gly Pro Glu Thr Ala Pro Pro
65                  70                  75                  80

Pro Pro Glu Ala Leu His Ser Leu Pro Gly Val Ser Leu Ser Leu Glu
                85                  90                  95

Asn Gln Glu Leu Trp Lys Glu Phe Ser Ala Val Gly Thr Glu Met Ile
            100                 105                 110

Ile Thr Lys Ala Gly Arg Arg Met Phe Pro Ala Cys Arg Val Ser Val
        115                 120                 125

Thr Gly Leu Asp Pro Glu Ala Arg Tyr Leu Phe Leu Leu Asp Val Val
    130                 135                 140

Pro Val Asp Gly Ala Arg Tyr Arg Trp Gln Gly Gln His Trp Glu Pro

145                 150                 155                 160

Ser Gly Lys Ala Glu Pro Arg Leu Pro Asp Arg Val Tyr Ile His Pro
                165                 170                 175

Asp Ser Pro Ala Thr Gly Ala His Trp Met Arg Gln Pro Val Ser Phe
            180                 185                 190

His Arg Val Lys Leu Thr Asn Ser Thr Leu Asp Pro His Gly His Leu
        195                 200                 205

Ile Leu His Ser Met His Lys Tyr Gln Pro Arg Ile His Leu Val Arg
    210                 215                 220

Ala Thr Gln Leu Cys Ser Gln His Trp Gly Gly Val Ala Ser Phe Arg
225                 230                 235                 240

Phe Pro Glu Thr Thr Phe Ile Ser Val Thr Ala Tyr Gln Asn Pro Arg
                245                 250                 255

Ile Thr Gln Leu Lys Ile Ala Ala Asn Pro Phe Ala Lys Gly Phe Arg
            260                 265                 270

Glu Asn Gly Arg Asn Cys Lys Arg Glu Arg Asp Ala Arg Val Lys Arg
        275                 280                 285

Lys Leu Arg Gly Pro Glu Pro Val Ala Thr Glu Ala Cys Gly Ser Gly
    290                 295                 300

Asp Thr Pro Gly Gly Pro Cys Asp Ser Thr Leu Gly Gly Asp Ile Arg
305                 310                 315                 320

Asp Ser Asp Pro Glu Gln Ala Pro Thr Pro Gln Glu Ala Ala Ser Ala
                325                 330                 335

Ser Ala Pro Pro Cys Gly Gly Pro Ser Ala Glu Ala Tyr Leu Leu His
            340                 345                 350

Pro Ala Ala Phe His Gly Ala Pro Ser His Leu Pro Ala Arg Thr Pro
        355                 360                 365

Ser Phe Ala Glu Ala Pro Asp Pro Gly Arg Pro Ala Pro Tyr Ser Ala
    370                 375                 380

Ala Phe Leu Asp Leu Gln Pro Gly Pro Gly Gly Ser Ala Tyr Gln Ala
385                 390                 395                 400

Ala Pro Ser Val Pro Ser Phe Ala Pro His Phe Ile Gln Gly Gly Pro
                405                 410                 415

Phe Pro Leu Pro Tyr Pro Gly Pro Gly Gly Tyr Leu Asp Met Gly Ser
            420                 425                 430

Lys Pro Met Tyr
        435

<210>  5
<211>  4230
<212>  DNA
<213>  Homo sapiens

<400>  5
gaagggtcgg gggatccctc cgccgccagc gcgtggtccc ggccccctcc acccgccgtc      60

tcggccgcgg ccagcagccc ctgccccccg ggggacgctg acggccgccc ggcgcgccgc     120

cctagcagac ggacaggggg cgctgcgcgc ggcctggggc aacccgggcc acaggggcag     180

gaaagtgagg gcccaggtcg gcccgggcgt gcaggggccc cgggttcgca gcggcggccg     240

cggcagcgat agcggcacta gcagcagcgg gagtgccggg ttgagccggg aagccgatgg     300

cggcggctgc ggcggctccg attcctcgct gactgcccgt ccgccctcct gcatcgagcg     360

ccatgttacc gacccaagct ggggccgcgg cggctctggg ccggggctcg gccctggggg     420

gcagcctgaa ccggaccccg acggggcggc cgggcggcgg cggcgggaca cgcggggcta     480

acgggggccg ggtccccggg aatggcgcgg ggctcgggcc cggccgcctg gagcgggagg     540

ctgcggcagc ggcggcaacc accccggcgc ccaccgcggg ggccctctac agcggcagcg     600

agggcgactc ggagtcgggc gaggaggagg agctgggcgc cgagcggcgc ggcctgaagc     660

ggagcctgag cgagatggag atcggtatgg tggtcggtgg gcccgaggcg tcggcagcgg     720

ccaccggggg ctacggcccg gtgagcggcg cggtgagcgg ggccaagccg ggtaagaaga     780

cccggggccg cgtgaagatc aagatggagt tcatcgacaa caagctgcgg cgctacacga     840

ccttcagcaa gaggaagacg ggcatcatga agaaggccta tgagctgtcc acgctgacag     900

ggacacaggt gctgttgctg gtggccagtg agacaggcca tgtgtatacc tttgccaccc     960

gaaaactgca gcccatgatc accagtgaga ccggcaaggc actgattcag acctgcctca    1020

actcgccaga ctctccaccc cgttcagacc ccacaacaga ccagagaatg agtgccactg    1080

gctttgaaga gacagatctc acctaccagg tgtcggagtc tgacagcagt ggggagacca    1140

aggacacact gaagccggcg ttcacagtca ccaacctgcc gggtacaacc tccaccatcc    1200

aaacagcacc tagcacctct accaccatgc aagtcagcag cggcccctcc tttcccatca    1260

ccaactacct ggcaccagtg tctgctagtg tcagccccag tgctgtcagc agtgccaatg    1320

ggactgtgct gaagagtaca ggcagcggcc ctgtctcctc tgggggcctt atgcagctgc    1380

ctaccagctt caccctcatg cctggtgggg cagtggccca gcaggtccca gtgcaggcca 1440

ttcaagtgca ccaggcccca cagcaagcgt ctccctcccg tgacagcagc acagacctca 1500

cgcagacctc ctccagcggg acagtgacgc tgcccgccac catcatgacg tcatccgtgc 1560

ccacaactgt gggtggccac atgatgtacc ctagcccgca tgcggtgatg tatgccccca 1620

cctcgggcct gggtgatggc agcctcaccg tgctgaatgc cttctcccag gcaccatcca 1680

ccatgcaggt gtcacacagc caggtccagg agccaggtgg cgtcccccag gtgttcctga 1740

cagcatcatc tgggacagtg cagatccctg tttcagcagt tcagctccac cagatggctg 1800

tgatagggca gcaggccggg agcagcagca acctcaccga gctacaggtg gtgaacctgg 1860

acaccgccca cagcaccaag agtgaatgat ccgcccgccg ccctggacag atggcccaag 1920

ggatggcacc acttatttat tgttgccttt tcacgttttc tttacacaca cgttgacggg 1980

ccgcaggagg gaggcgggga ggaggaacgg gcagccacag gactgagccc tctcactcca 2040

gccaaagaaa tgggcctgcc tgcctccacc cgtcctccct cagcctcccc ttcttcccgc 2100

cccacctccc atttctgttg ctggaggggc tgtcctcctt cctgggaccc cctcgccagc 2160

ttggctcgat gtttgccatg agtattagct tacccaatgg gaccgtgccc cacctcccca 2220

cacacaggcc ttctgtgggg ctgggcaccg tgtcctcctc tgaggaagca gttgggggccc 2280

tcttgccagc ctccttgctg accccaggtc agccctgtgt ctgtcacagg ctgggtcaaa 2340

agagccctgg ctctgcccct cagggggcca gctggggaga tgggggcttc ttcctcacac 2400

tgctgtcctc tcccccttca gctcctgagt agctgggcct gtgcactggg caggttcctg 2460

gggccgcctg ccctgccttg ccgctcccct tggacctcca ggggctcctg ggttggaggg 2520

aaccaccagc gttcccttct cccccttgtc ttcccccctc tcctcccagc tgctttactt 2580

aaagttgatt ttgaactttt tatttgagga gacgaagtga aaacaaatct ataaatatat 2640

atttttaaaa tatttaactt ttttttatgg cgtttttctc gtccccctcc ctgcccaaac 2700

tccccttccc tggggagccc tcaggctccc cagaactggc tgggcccctg gggacagagc 2760

caccccatga gctcggggtc caccagtgtg tgggggagat tctgggtttg cccagtcctg 2820

ggttgtttcc aggagaaagc cgggggaggg gccctcaggc cattccccaa cggggtgggg 2880

agggtgaccc acagctctgg gcctcttttt gccctttagg gctgttgcta gggagaggga 2940

agagggagac caaatgtcgg ggttggggtg ggagggcgtc aggcagaggc aactgacttc 3000

atttgtgcca cacgcatggg cattgcagcc ttgcgctgtc ccaggcatgc agctgcctgg 3060

ggcccaagtt gcagtgagca gggtggggtc tgggaggggg tgagaggcag gaatgggggt 3120

cagaagaagt gggagcagct tcttgggctg agtgcagcca aaggggagcc agaaatgggc 3180

agttctccca gggagtgagc agctactgta acttttttaa attaagacaa aaagccttga 3240

agaaaatgac tttatttttc taagtgtaac ctcagtattt atgtaatttg tacaggggcc 3300

atgccccacc cccctcctcc ccctttgggg tagaccttga gggtgggcca gcataggggg 3360

gagggtcttt taccctgtgt cagagcctac cttcaccacc tatatccaga aggggagctt 3420

tttcagaaac agggcagcag tggggtgaaa ttttcttaac ccctaagact gccttcagta 3480

ggaacaagct ggcttctgtg attaggtgaa gggatggggg aagattttat gcacagccta 3540

gttatcaagg ggatgatttg ccgacatgtt tgagaacccc ctaacctcta accctcattg 3600

ctgtcttgcc ccagtttggg gtgccaagat ggaagtcacc tttctgggct ttctcctgga 3660

gatagctggg gcttatgggt ggctttcaag gctggggcat ggcaaatcag gggccagaga 3720

gcaggggagc ttgggactca ggtctgtaac tgcccagccc cttttctctg ctcttgtttc 3780

actccaccat cactcactca ctccccactc ccccacccat ggggaggaga cctttgatga 3840

attcttcctc tccttcccac aaaagacaga cccagtgagt gaatcaggca aagtgcttat 3900

aatgtgtgtt gtgtgagcgt ggccttggga ggacatgcgt gtgtcaggga tgagttgagg 3960

tgatattttt atgtgcagcg acccttggtg tttcccttcc tcggtggctc tggggtatgt 4020

gtgtgtgggt gtgtgcgcct gagtgagtgt gtgtgcttga atgtgagtgt gtatgtcagt 4080

ggtttctact tcccctggga tgctgaccca ggaatagtgg acatggtcac agtcctatgt 4140

acagagcttt cttttgtatt aaaaaaaaat actctttcaa taaatgtatc atttttgtgc 4200

acagaaaaaa aaaaaaaaaa aaaaaaaaaa 4230

<210> 6
<211> 508
<212> PRT
<213> Homo sapiens

<400> 6

Met Leu Pro Thr Gln Ala Gly Ala Ala Ala Ala Leu Gly Arg Gly Ser
1               5                   10                  15

Ala Leu Gly Gly Ser Leu Asn Arg Thr Pro Thr Gly Arg Pro Gly Gly
            20                  25                  30

Gly Gly Gly Thr Arg Gly Ala Asn Gly Gly Arg Val Pro Gly Asn Gly
        35                  40                  45

Ala Gly Leu Gly Pro Gly Arg Leu Glu Arg Glu Ala Ala Ala Ala Ala
    50                  55                  60

Ala Thr Thr Pro Ala Pro Thr Ala Gly Ala Leu Tyr Ser Gly Ser Glu
65                  70                  75                  80

Gly Asp Ser Glu Ser Gly Glu Glu Glu Glu Leu Gly Ala Glu Arg Arg

                85                  90                  95

Gly Leu Lys Arg Ser Leu Ser Glu Met Glu Ile Gly Met Val Val Gly
            100                 105                 110

Gly Pro Glu Ala Ser Ala Ala Ala Thr Gly Gly Tyr Gly Pro Val Ser
        115                 120                 125

Gly Ala Val Ser Gly Ala Lys Pro Gly Lys Lys Thr Arg Gly Arg Val
    130                 135                 140

Lys Ile Lys Met Glu Phe Ile Asp Asn Lys Leu Arg Arg Tyr Thr Thr
145                 150                 155                 160

Phe Ser Lys Arg Lys Thr Gly Ile Met Lys Lys Ala Tyr Glu Leu Ser
                165                 170                 175

Thr Leu Thr Gly Thr Gln Val Leu Leu Leu Val Ala Ser Glu Thr Gly
            180                 185                 190

His Val Tyr Thr Phe Ala Thr Arg Lys Leu Gln Pro Met Ile Thr Ser
        195                 200                 205

Glu Thr Gly Lys Ala Leu Ile Gln Thr Cys Leu Asn Ser Pro Asp Ser
    210                 215                 220

Pro Pro Arg Ser Asp Pro Thr Thr Asp Gln Arg Met Ser Ala Thr Gly
225                 230                 235                 240

Phe Glu Glu Thr Asp Leu Thr Tyr Gln Val Ser Glu Ser Asp Ser Ser
                245                 250                 255

Gly Glu Thr Lys Asp Thr Leu Lys Pro Ala Phe Thr Val Thr Asn Leu
            260                 265                 270

Pro Gly Thr Thr Ser Thr Ile Gln Thr Ala Pro Ser Thr Ser Thr Thr
        275                 280                 285

Met Gln Val Ser Ser Gly Pro Ser Phe Pro Ile Thr Asn Tyr Leu Ala
    290                 295                 300

Pro Val Ser Ala Ser Val Ser Pro Ser Ala Val Ser Ser Ala Asn Gly
305                 310                 315                 320

Thr Val Leu Lys Ser Thr Gly Ser Gly Pro Val Ser Ser Gly Gly Leu
                325                 330                 335

Met Gln Leu Pro Thr Ser Phe Thr Leu Met Pro Gly Gly Ala Val Ala
            340                 345                 350

Gln Gln Val Pro Val Gln Ala Ile Gln Val His Gln Ala Pro Gln Gln
        355                 360                 365

Ala Ser Pro Ser Arg Asp Ser Ser Thr Asp Leu Thr Gln Thr Ser Ser
    370                 375                 380

Ser Gly Thr Val Thr Leu Pro Ala Thr Ile Met Thr Ser Ser Val Pro
385                 390                 395                 400

Thr Thr Val Gly Gly His Met Met Tyr Pro Ser Pro His Ala Val Met
                405                 410                 415

Tyr Ala Pro Thr Ser Gly Leu Gly Asp Gly Ser Leu Thr Val Leu Asn
            420                 425                 430

Ala Phe Ser Gln Ala Pro Ser Thr Met Gln Val Ser His Ser Gln Val
        435                 440                 445

Gln Glu Pro Gly Gly Val Pro Gln Val Phe Leu Thr Ala Ser Ser Gly
    450                 455                 460

Thr Val Gln Ile Pro Val Ser Ala Val Gln Leu His Gln Met Ala Val
465                 470                 475                 480

Ile Gly Gln Gln Ala Gly Ser Ser Ser Asn Leu Thr Glu Leu Gln Val
                485                 490                 495

Val Asn Leu Asp Thr Ala His Ser Thr Lys Ser Glu
            500                 505

<210>  7
<211>  4093
<212>  DNA
<213>  Mus musculus

<400>  7
   agccagcgcg tggtcccggc cccctccacc cgcggtctcg gccgcggcca gcagcccctg      60
   ccccgcgggg gacgctgacg gccgcagggc gcgcctcccc ggcacacgga cagggggcgc     120
   tgcgcgcggc ctggggcaac cccggccata ggggcaggaa agtgagggcc caggtcggcc     180
   cgggcgtgca ggggccccgg gctcgcagcg gcggccgcgg cagcaagagc ggctttagta     240
   acagcgcgag tgccgggtgg aaccgagaag ccgatggcgg cagctgcccc gattcctcgc     300
   tgacttgccc gtcagccctc ctgcactgag cgccatgtta ccgagccaag ctggggccgc     360
   ggcggctctg ggccggggct cggccctggg gggcaacctg aaccggaccc cgacggggcg      420
   gccgggcggc ggcggcggga ctcgcggggc gaacgggggc cgggtccccg ggaacggcgc 480
   ggggctcggc cagagtcgtc tggagcggga ggctgcagcg gcagcggcgc ccaccgccgg 540
   ggccctctac agcggcagcg agggcgactc cgagtccggc gaggaggagg agctgggcgc 600
   cgagcggcgc ggcctcaagc ggagcctgag cgagatggag ctcggcgtgg tggtcggtgg 660
   gcctgaggcg gcggcggcgg ccgccggggg ctacggcccg gtgagcggcg cggtgagcgg 720
   ggccaagccg gggaagaaga cccggggccg cgtgaagatc aagatggagt tcatcgacaa 780
   caagctgcgg cgctacacga ccttcagcaa gaggaagacg ggcatcatga agaaggccta 840
   tgagctgtcc acgctgacag ggacacaggt gctgttgctg gtggccagtg agacaggcca 900
   tgtgtatacc tttgccaccc gcaaactgca gcccatgatc accagtgaga ccggcaaggc 960
   gctgattcag acctgcctca actcgccaga ctctccgccc cgctcagacc ccaccacaga 1020
   ccagagaatg agtgccactg gctttgaaga gccagatctc acctaccagg tgtcggaatc 1080
   tgacagcagt ggggaaacca aggacacact gaagccagca ttcacagtca ccaacctgcc 1140
   gggtaccacc tccacaatcc agacagcacc cagcacctct accaccatgc aagtcagcag 1200
   cggcccctcc ttccccatca ccaactacct ggcaccagtg tctgctagtg tcagccccag 1260
   cgctgtcagc agtgccaacg ggactgtgct caagagtaca ggcagcggcc ctgtctcctc 1320
   tgggggcctt atgcagctgc ctaccagctt caccctcatg cctggtgggg cagtggccca 1380
   gcaggtccct gtgcaggcca ttcatgtgca ccaggcccca cagcaagcgt ctccctctcg 1440
   tgacagcagc acagacctca cgcagacctc ctccagcggg acagtgacgt tgcccgccac 1500
   catcatgacg tcgtctgtac ccacaactgt gggtggccac atgatgtacc ctagtcccca 1560
   tgcagtgatg tatgccccca cctcaggcct ggctgatggc agcctcaccg tgctcaatgc 1620
   cttctctcag gcaccatcca ccatgcaggt gtcccacagc caggtccagg agccaggtgg 1680
   tgtccctcag gtgttcctga cagcaccgtc tgggaccgtg cagatccctg tctctgcagt 1740
   tcagcttcac cagatggctg tgatagggca gcaagctggg agcagcagca acctcaccga 1800
   gctacaggtg gtgaacctgg atgccaccca cagcaccaag agtgaatgat ccgcccgtca 1860
   ccctggacag acggcccaag ggacggcacc acttatttat tgttgccttt tcacggtttc 1920
   tttacacaca cactgactgg ccgaaggagg gaggcgggga gaaggagcgg gcagccacag 1980
   gactatgccc tctcactcca gccaaagaaa cgggcctgcc tgccctcccc agctgccctc 2040
   cttcctgctc tgcctgtcat gctgttgatg gtgcagctct cctccctcct cagaccccct 2100
   gccagcctgg ctcaatttgc tatgagtatt agctgacccg atgggactgt gccaccttcc 2160
   cactcacagg ccttctgtgg gactgggcac agtgttcccg tccgaggaaa catttggggc 2220
   cctctctcca gcctctttgg cgacctctgg ttagctttgt gtctgccatg gtctgggtca 2280
   aaagagcccg gcccctgccc ctcagggagc cagctgggag agatatgggg gcttgtgccc 2340
   tcagccgatg ccctctgccc ctttcagctc ctgaagcagc tgggcctgtg caccgggcag 2400
   gttactgggg ccgcctgccc tgccttgcct ctgttcccgc ggacctgcag gggctcctgg 2460
   gttggaggga accactgtta gcggtctctt cctccccttg gtcttccccc ttctcccagc 2520
   tgctttactt aagagttgat tttgaacttt ttatttgagg agatgacgtg aaaacaaatc 2580
   tataaatata tatttttaaa atatttaact tttttttaat ggcgtttttc tcatccccct 2640
   ccctgcccaa actcctcttg gggaagctct tgggctccct agaactgtct gggcccctgg 2700
   ggacagccac cccatgagct tggggtccac cagtgtgtgt gtgtgggaga ttctgggatt 2760
   gctccacatc ctgggttctt tccaggagaa aggtggggga ggggccctca ggccaagcca 2820
   cagaaggggg atgggaggtc ccgcagttct aggcctcttt tgccctctag ggttgttgct 2880
   aggaaaaggg aagatggaga ccaaatgtgg gggtggggtg ggaggggtca gggagaggag 2940
   attgatgtcc tttgtgccac agtatggtcg ttgcagcctg tgctccccca ggcttgcaga 3000
   tacctggggc ccaaggtgca gtgggcagga tggagttcgg gaggtaggga gatacaggaa 3060
   tgggggtcag aggtaaaagc tcttgggctg agtgcagcaa tgggggccca gaggtgggca 3120
   gctctcccag ggagtgagca gctactgtaa cttttttaaa ttaagacaaa aagccttgaa 3180
   gaaaatgact ttatttttct aagtgtaacc tcagtattta tgtaatttgt acagggccat 3240
   gccccatccc ttaaaatccc tttggggacc ttgagggtgg gctagcatag gggagggtgt 3300
   tttaccctgt gtcagagcca gcctaaacca cctgtatcca ttaaaggagc tttttccggg 3360
   agggcagttg gggtagggtg tcactaaacc cttgttgctg ccttcagtag gaacaagcct 3420
   acttctgcgg ttaggtggag gggttgggaa ggttttatgc acagcctacg tttcatgggg 3480
   atgatctgct gacgtttaag agccccctga acctctaatc cccattactg tcttccccag 3540
   ctgggatgcc acctttctgg cctttctcct ggaaatagct ggggcgtttg ggtggctttc 3600
   aagactgggg catggtaaac caggggtcag agttggggag cttgggactc aggtctgtaa 3660
   ctgcccagcc cccttctctg ctcctgtttc actcctctgc cgcccactca tggcccctcc 3720
   cccaacaccg gaggtgttga ttcctcctca caaaagatag agcctgggca agtgattcag 3780
   gccaagtgct taccaacgtg tgctgtgtga ttgtggcctt gggagaacct gtttgtcagg 3840
   caggactgag gtggcatttc tatgtgcagc aacccttggt gtatccctaa ttaagtggct 3900
   ctaggggtgt gtgcgcgcgc ctgtgtccga gtgaatacgt gtgtgaatgt ggagtgcgtg 3960
   tggtatatcc gtggtttcta cttctcctgg gatgttgacc caggaatagt ggacatggtc 4020
   acatcttctg tacagagctt tcttttgtat taaaaaaata ctctttcaat aaatgtatca 4080
   tttttgtgca cag 4093

<210>  8
<211>  504
<212>  PRT
<213>  Mus musculus

<400>  8

Met Leu Pro Ser Gln Ala Gly Ala Ala Ala Ala Leu Gly Arg Gly Ser
1               5                   10                  15

Ala Leu Gly Gly Asn Leu Asn Arg Thr Pro Thr Gly Arg Pro Gly Gly
            20                  25                  30

Gly Gly Gly Thr Arg Gly Ala Asn Gly Gly Arg Val Pro Gly Asn Gly
        35                  40                  45

Ala Gly Leu Gly Gln Ser Arg Leu Glu Arg Glu Ala Ala Ala Ala Ala
    50                  55                  60

Ala Pro Thr Ala Gly Ala Leu Tyr Ser Gly Ser Glu Gly Asp Ser Glu
65                  70                  75                  80

Ser Gly Glu Glu Glu Glu Leu Gly Ala Glu Arg Arg Gly Leu Lys Arg
                85                  90                  95

Ser Leu Ser Glu Met Glu Leu Gly Val Val Val Gly Gly Pro Glu Ala
            100                 105                 110

Ala Ala Ala Ala Ala Gly Gly Tyr Gly Pro Val Ser Gly Ala Val Ser
        115                 120                 125

Gly Ala Lys Pro Gly Lys Lys Thr Arg Gly Arg Val Lys Ile Lys Met
    130                 135                 140

Glu Phe Ile Asp Asn Lys Leu Arg Arg Tyr Thr Thr Phe Ser Lys Arg
145                 150                 155                 160

Lys Thr Gly Ile Met Lys Lys Ala Tyr Glu Leu Ser Thr Leu Thr Gly
                165                 170                 175

Thr Gln Val Leu Leu Leu Val Ala Ser Glu Thr Gly His Val Tyr Thr
            180                 185                 190

Phe Ala Thr Arg Lys Leu Gln Pro Met Ile Thr Ser Glu Thr Gly Lys
        195                 200                 205

Ala Leu Ile Gln Thr Cys Leu Asn Ser Pro Asp Ser Pro Pro Arg Ser
    210                 215                 220

Asp Pro Thr Thr Asp Gln Arg Met Ser Ala Thr Gly Phe Glu Glu Pro
225                 230                 235                 240

Asp Leu Thr Tyr Gln Val Ser Glu Ser Asp Ser Ser Gly Glu Thr Lys
                245                 250                 255

Asp Thr Leu Lys Pro Ala Phe Thr Val Thr Asn Leu Pro Gly Thr Thr
            260                 265                 270

Ser Thr Ile Gln Thr Ala Pro Ser Thr Ser Thr Thr Met Gln Val Ser
        275                 280                 285

Ser Gly Pro Ser Phe Pro Ile Thr Asn Tyr Leu Ala Pro Val Ser Ala
    290                 295                 300

Ser Val Ser Pro Ser Ala Val Ser Ser Ala Asn Gly Thr Val Leu Lys
305                 310                 315                 320

Ser Thr Gly Ser Gly Pro Val Ser Ser Gly Gly Leu Met Gln Leu Pro
                325                 330                 335

Thr Ser Phe Thr Leu Met Pro Gly Gly Ala Val Ala Gln Gln Val Pro
            340                 345                 350

Val Gln Ala Ile His Val His Gln Ala Pro Gln Gln Ala Ser Pro Ser
        355                 360                 365

Arg Asp Ser Ser Thr Asp Leu Thr Gln Thr Ser Ser Ser Gly Thr Val
    370                 375                 380

Thr Leu Pro Ala Thr Ile Met Thr Ser Ser Val Pro Thr Thr Val Gly
385                 390                 395                 400

Gly His Met Met Tyr Pro Ser Pro His Ala Val Met Tyr Ala Pro Thr
                405                 410                 415

Ser Gly Leu Ala Asp Gly Ser Leu Thr Val Leu Asn Ala Phe Ser Gln
            420                 425                 430

Ala Pro Ser Thr Met Gln Val Ser His Ser Gln Val Gln Glu Pro Gly
        435                 440                 445

Gly Val Pro Gln Val Phe Leu Thr Ala Pro Ser Gly Thr Val Gln Ile
    450                 455                 460

Pro Val Ser Ala Val Gln Leu His Gln Met Ala Val Ile Gly Gln Gln
465                 470                 475                 480

Ala Gly Ser Ser Ser Asn Leu Thr Glu Leu Gln Val Val Asn Leu Asp
                485                 490                 495

Ala Thr His Ser Thr Lys Ser Glu
            500

<210>  9
<211>  8580
<212>  DNA
<213>  Homo sapiens

<400>  9
   aatcgccggc agcctatgac atcagacagg aacgcctggg atgccgcgct gctcctggcc      60
   aacctccgag gaggaggagg gtcccgccgg ctaagagtta attagccccg cacggcgagg     120
   ggggaggcgc cagttttctg gggacactgg ctgccactgt actcctaccc aggggagctc     180
   acggagagtt ggatgaattc tgggttgtta gctgcggtca gctgggctcc cgggagcctg     240
   ttgctggtgg agaacagggg gcgcctggcc aagggaccag cggcttgctg agactcaaca     300
   tgacactcct ggggtctgag cattccttgc tgattaggag caagttcaga tcagttttac     360
   agttaagact tcaacaaaga aggacccagg aacaactggc taaccaaggc ataataccac     420
   cactgaaacg tccagctgaa ttccatgagc aaagaaaaca tttggatagt gacaaggcta     480
   aaaattccct gaagcgcaaa gccagaaaca ggtgcaacag tgccgacttg gttaatatgc     540
   acatactcca agcttccact gcagagaggt ccattccaac tgctcagatg aagctgaaaa     600
   gagcccgact cgccgatgat ctcaatgaaa aaattgctct acgaccaggg ccactggagc     660
   tggtggaaaa aaacattctt cctgtggatt ctgctgtgaa agaggccata aaaggtaacc     720
   aggtgagttt ctccaaatcc acggatgctt ttgcctttga agaggacagc agcagcgatg     780
   ggctttctcc ggatcagact cgaagtgaag acccccaaaa ctcagcggga tccccgccag     840
   acgctaaagc ctcagatacc ccttcgacag gttctctggg gacaaaccag gatcttgctt     900
   ctggctcaga aaatgacaga aatgactcag cctcacagcc cagccaccag tcagatgcgg     960
   ggaagcaggg gcttggcccc cccagcaccc ccatagccgt gcatgctgct gtaaagtcca    1020
   aatccttggg tgacagtaag aaccgccaca aaaagcccaa ggaccccaag ccaaaggtga    1080
   agaagcttaa atatcaccag tacattcccc cagaccagaa ggcagagaag tcccctccac    1140
   ctatggactc agcctacgct cggctgctcc agcaacagca gctgttcctg cagctccaaa    1200
   tcctcagcca gcagcagcag cagcagcaac accgattcag ctacctaggg atgcaccaag    1260
   ctcagcttaa ggaaccaaat gaacagatgg tcagaaatcc aaactcttct tcaacgccac    1320
   tgagcaatac ccccttgtct cctgtcaaaa acagtttttc tggacaaact ggtgtctctt    1380
   ctttcaaacc aggcccactc ccacctaacc tggatgatct gaaggtctct gaattaagac    1440
   aacagcttcg aattcggggc ttgcctgtgt caggcaccaa aacggctctc atggaccggc 1500
   ttcgaccctt ccaggactgc tctggcaacc cagtgccgaa ctttggggat ataacgactg 1560
   tcacttttcc tgtcacaccc aacacgctgc ccaattacca gtcttcctct tctaccagtg 1620
   ccctgtccaa cggcttctac cactttggca gcaccagctc cagccccccg atctccccag 1680
   cctcctctga cctgtcagtc gctgggtccc tgccggacac cttcaatgat gcctccccct 1740
   ccttcggcct gcacccgtcc ccagtccacg tgtgcacgga ggaaagtctc atgagcagcc 1800
   tgaatggggg ctctgttcct tctgagctgg atgggctgga ctccgagaag gacaagatgc 1860
   tggtggagaa gcagaaggtg atcaatgaac tcacctggaa actccagcaa gagcagaggc 1920
   aggtggagga gctgaggatg cagcttcaga agcagaaaag gaataactgt tcagagaaga 1980
   agccgctgcc tttcctggct gcctccatca agcaggaaga ggctgtctcc agctgtcctt 2040
   ttgcatccca agtacctgtg aaaagacaaa gcagcagctc agagtgtcac ccaccggctt 2100
   gtgaagctgc tcaactccag cctcttggaa atgctcattg tgtggagtcc tcagatcaaa 2160
   ccaatgtact ttcttccaca tttctcagcc cccagtgttc ccctcagcat tcaccgctgg 2220
   gggctgtgaa aagcccacag cacatcagtt tgcccccatc acccaacaac cctcactttc 2280
   tgccctcatc ctccggggcc cagggagaag ggcacagggt ctcctcgccc atcagcagcc 2340
   aggtgtgcac tgcacagaac tcaggagcac acgatggcca tcctccaagc ttctctcccc 2400
   attcttccag cctccacccg cccttctctg gagcccaagc agacagcagt catggtgccg 2460
   gggggaaccc ttgtcccaaa agcccatgtg tacagcaaaa gatggctggt ttacactctt 2520
   ctgataaggt ggggccaaag ttttcaattc catccccaac tttttctaag tcaagttcag 2580
   caatttcaga ggtaacacag cctccatcct atgaagatgc cgtaaagcag caaatgaccc 2640
   ggagtcagca gatggatgaa ctcctggacg tgcttattga aagcggagaa atgccagcag 2700
   acgctagaga ggatcactca tgtcttcaaa aagtcccaaa gatacccaga tcttcccgaa 2760
   gtccaactgc tgtcctcacc aagccctcgg cttcctttga acaagcctct tcaggcagcc 2820
   agatcccctt tgatccctat gccaccgaca gtgatgagca tcttgaagtc ttattaaatt 2880
   cccagagccc cctaggaaag atgagtgatg tcacccttct aaaaattggg agcgaagagc 2940
   ctcactttga tgggataatg gatggattct ctgggaaggc tgcagaagac ctcttcaatg 3000
   cacatgagat cttgccaggc cccctctctc caatgcagac acagttttca ccctcttctg 3060
   tggacagcaa tgggctgcag ttaagcttca ctgaatctcc ctgggaaacc atggagtggc 3120
   tggacctcac tccgccaaat tccacaccag gctttagcgc cctcaccacc agcagcccca 3180
   gcatcttcaa catcgatttc ctggatgtca ctgatctcaa tttgaattct tccatggacc 3240
   ttcacttgca gcagtggtag aatgcccaat gcaccagtgc tatggaagac caatggagtt 3300
   ccatggggga aagcacacag ccatacatac tttactgtcc aaaaacagaa gaagaagaag 3360
   agaattaaaa agaagcaatg atttctgtgc caatgaacaa gaacaaaagt catttttaga 3420
   aatacatata ctgtaatatt taccaacagt cagtaactgt taatgatttc aacaatgcat 3480
   taaaagaatg tgctttctca gattaaggat gccaaaaaag atatttcact gccttttcaa 3540
   agaccagtat attttctagc ccataatttt tctcaggcat tgttggggca taagctcaca 3600
   ctgtaagctt ttctcatgaa ttcactagac ataacgtgga aggaaaacgt agtcttttgg 3660
   gagtacaggg aagccagccc ctcaaagctt atggaagaca tacctgcaat ggaagctgtt 3720
   gcccaatgtc tccattacta tctttcaaaa gagaagccag acccagcttc agatcaaaag 3780
   ttcttgagac agaggaacaa aaccaatcga tttccaggga agctaatcaa ctctcttttc 3840
   cctctaccac aaaactgccc tgctggagtg gttctgaacc tgtacccagg actcgatgtg 3900
   gtcactaata acaattaacc tgaactgagt ccacagaact ccactcggaa ctttcttctt 3960
   ttttaactag tggcccaatc attcccacca tctctgtgct gataagtacg tgtcctagat 4020
   gagaaccctg aagaatgcag accttcttcc cccgaaggag atgccacaag ctctccaaca 4080
   cagccccctt tagttccaaa gactagagat gaccacattg gtagaagtat atctcgaggc 4140
   acaggaaggg agccccacca gggataattc agacaggact agagaataac atcatttcac 4200
   ataccctggg ataaacaccc tgggttccta tagaaggact attacttatg ggagtccaac 4260
   ttctcctttt gttttgttat tatcagttta tctttctccc actccacttt tccttcaagg 4320
   taccaatcct ttcctgttcc tcgtttggcc atctttcttt ttctgcctcc acattgggag 4380
   gggaggactt ctcagttcta acaagctgcc atactcctaa gaaagccatt tttgaaaaat 4440
   ttaacaatcc aggttcttct ggagaactca ttctccacac gcacagtttg ctgcaaaagg 4500
   aagttgcaag aatttcttga ggaagaaact ggtgacttgg tccatcagtc acgaagttct 4560
   ttctattctc gtttagtttt caagaaatta ttggtttgtg ttgctctggg gaaattggaa 4620
   atcattacat tgtaaagaca aatatggatg atatttacaa gagagaattt cagatctggg 4680
   tttttgaaag aaaacagaat tgcgcattga aaacgatgga aggaaaaaga caatggtcta 4740
   atgtgcattc ctcattacct ctcgtggctt tggctgggag ttggaaaaag ctaaaatttc 4800
   agaacagtct ctgtaaggct ctctgtggct ccagttcacc attttatatt gttgcatgct 4860
   gtagaaagga gctattgctg ttgttttgtt tttttattta aatcactaag gcactgtttt 4920
   tatcttttgt aaaaaaaaaa aaaaagttgt tcactgtgca cttatagaaa aaataatcaa 4980
   aaatgttggg attttagaag ctctcttttt gataaaccaa agatttagaa gtcattccat 5040
   tgttaacttg taaaaatgtg tgaacacaga gagtttttgg tgattgctac tctgaaagct 5100
   gccagatctt attctggggg tgggatgtgg aggaatacac atacacacac aaacatacat 5160
   gtatgtataa tagatatata catatgtgta tattatatct gtgtgtgcat gtatctccaa 5220
   aagcggcgtt acagagttct acaccaaaag cctttaaccc ttaatctgct gtgaatgata 5280
   cctggccttt ctcactatga atttctgatt aaccaaccag actacacgtt gcctctctgt 5340
   gtatgactaa cggctccaac ccgatgactc acagctactt gcttatcgtg aacaagctca 5400
   tcttggcaat gaatatggat gtgaaaagac agaacagctt caccattagt agctggaaat 5460
   ggtatcacag tctcttatag aggaatatga aaggaacaag aaaatcattt tacattcctt 5520
   ttatctgtat tgtgctttaa aagatccaca tggtaaattt tttattttgc ttttatgtca 5580
   gtcatcagaa ccaaaaaaat ccagaagaaa aaattgccag tgtttccttt gaagatgaag 5640
   ctactgggga agaaaacctt attaatacac tccacacatt tgttcattcc tcagctgttg 5700
   gtgttttctt ggggtcttga caaagcttgc tggtcagtgc acttttcagg tgtcacgttt 5760
   tgctgtttgt atgttttttc ttccccttac ttcctttgga aaacaaactc acacagtgcc 5820
   cctactctga gacctgggac tgagtgttaa ttattttttc cttgggtatt tctatctgag 5880
   agactagacc tagttaggag gcctctgtac ttctccagat tgtacctttt tatggggatc 5940
   tttgaggcta tgacccagga ctgatagata tgccttacgg aagacaaaag ataaaatggt 6000
   tcctatatcc taatgcaaac caacacagtt aaaagagcag atctctggat aactgctctc 6060
   aacctgcttc tacagtctcc acaaaccgca ttcaccctct ctcttcatag ctcagacatg 6120
   aaatttgagg gagaaaactg gagataattg ggagaaaatt gatgaagttg gctgcttcca 6180
   gtagatcaga taatccatga atttgtctcc cattgagaat tttattttaa attcttttaa 6240
   actcttcgtt gtgtcttttg tgatgacaaa tcaggcatga ctaaaagatg tacagagact 6300
   tacgaagatg gtcacattca agttccctaa tgctcttaga acctgaagat gaccatgtgt 6360
   agttttctta agacctctga acccccatgg tgatgaagac ttgaagacat ttgcagctat 6420
   ctgctgcagt ctggtagatt catacttatc taaagaagtc aaaaaattta ttcgtgcaag 6480
   tgcttgcagg aagccagtgc ttattagtag tgaccctgct tctatcaacg ttattgagac 6540
   aacacatatt ctattctaag ggagaaagag ggaggaagag agggagggag ggaggaagaa 6600
   gagggaggga gcgaggaagg aagataggag atgggtaggg gggtaaagag aaaaggaggg 6660
   agaagggaag gaaggaaaga agagaggaaa gaaaggaggg aaggaaaaaa gggccaaact 6720
   ttctgatcta tgaacttctc agttcagctg tcacattatg agaagtaaat cagaattttt 6780
   ttaaggagaa gtcattctta gcactacaat aattgtacca gtaattgagg aaaccaagac 6840
   aatcttcacc tgaataatag agggtctgag aactgtcagc cttttgccat tcaaaaacat 6900
   ttatgtccaa cctgaaaaaa aagcatcaat aaaacctatc ccaagcattc aaaatagtcc 6960
   tttccaaatg ttatttattt taaagtcaat cagctctttt agaaacagat tctggtctgg 7020
   ctgaaaactc ccacaacaaa tttactcatc cagtggctaa tatttaatgc ccaccatggg 7080
   cagagcacac aaatcttcag ataaacaata ctgaattgat tcagcacaga ttgttcagat 7140
   ttgaatgacc agggagttgt atttgcacat gcaagaacac taagaatctc ccagtcctca 7200
   aactagaaac ctttcagcta cgatgaaaaa aaaaaagggg tcttcatttt tccaagaggg 7260
   ggtggaggtg gggatcactt tttagctaaa agctatctct cacttcaaaa ttcttgtctt 7320
   tttctttgtg gacaaacacc agtagtctat cacttggaga tcttttaata tctcccatca 7380
   tttaaaacat ccacgagagt ttgaagattt gtgttgattg ccagatacag aagccccttg 7440
   aaaataagga aagggtggag gaagcatttt tgtgtcctat ccctacttat cgtagcagct 7500
   ctatagacaa aagggacact tactggtgag cctctggccc ttaaaagaaa atcatctaag 7560
   aatatgaagg caatttgatt tccccccaca gccctcagct gccttcctca cagaaggaag 7620
   ttcccaaaat tgctggtaca cagtttgcaa tcaaatatca gatatgagaa aacctgtagt 7680
   gaagagtctg ggttcttggt tttctcataa atccaatata aatttgtagg ttggttcagg 7740
   gtcaaaattg ccagtgcttt attagacaga tgatactgat agacacacag agcccaggtc 7800
   ctggaacaag acaatcctgt agtgccaaga tctggtcagt tgcgttaagg agctgggttt 7860
   gattctagag tccaggttta tagagaaacc ctggctagat tgagcctacc catggggaga 7920
   cgatttcaag acaggatgag atctgggaag aattttgttg tcatctgcca gggaaattat 7980
   cacaggactc attgaatgca ataacatgtg agtaagttcc cttttgattc tgggaatcag 8040
   cgattttccc tgtggattaa gacaaaccaa cgccagaagg tctcctgtgc ttattttaac 8100
   catctgctcc catcgtgaac cctggagcat gcatttccta gaagtggttt catagctcct 8160
   gtgtgttcat ggaaaagggg agtataatga tggggatgct ggaagctttt ttaatgtttt 8220
   ccaaaggaaa ggaacccaca ctgctcccca gagttccttt ccaatggccc tgcagtaaga 8280
   acggaggaca atgtattgct gggtgcttaa aatcctccct cagtgaagca caaagagaca 8340
   ctttgtaaag aaaaaaagag caagcatagg ttctctgtgg gaccttgtgg agtggtgttt 8400
   tcacgttggt ctctttggct caattgagca taatcagaaa gaaatgtggg ttattgggaa 8460
   gagacaaaaa gcagtggcta aaataccaaa gttggcatgt gttctttttt aaaaaaaaaa 8520
   aaaaatgcat atatttttaa ataaaatgtt tattttaaaa agaaaaaaaa aaaaaaaaaa 8580

<210> 10
<211> 986
<212> PRT
<213> Homo sapiens

<400> 10

Met Thr Leu Leu Gly Ser Glu His Ser Leu Leu Ile Arg Ser Lys Phe
1               5                   10                  15

Arg Ser Val Leu Gln Leu Arg Leu Gln Gln Arg Arg Thr Gln Glu Gln
            20                  25                  30

Leu Ala Asn Gln Gly Ile Ile Pro Pro Leu Lys Arg Pro Ala Glu Phe
        35                  40                  45

His Glu Gln Arg Lys His Leu Asp Ser Asp Lys Ala Lys Asn Ser Leu
    50                  55                  60

Lys Arg Lys Ala Arg Asn Arg Cys Asn Ser Ala Asp Leu Val Asn Met
65                  70                  75                  80

His Ile Leu Gln Ala Ser Thr Ala Glu Arg Ser Ile Pro Thr Ala Gln
                85                  90                  95

Met Lys Leu Lys Arg Ala Arg Leu Ala Asp Asp Leu Asn Glu Lys Ile
            100                 105                 110

Ala Leu Arg Pro Gly Pro Leu Glu Leu Val Glu Lys Asn Ile Leu Pro
        115                 120                 125

Val Asp Ser Ala Val Lys Glu Ala Ile Lys Gly Asn Gln Val Ser Phe
    130                 135                 140

Ser Lys Ser Thr Asp Ala Phe Ala Phe Glu Glu Asp Ser Ser Ser Asp
145                 150                 155                 160

Gly Leu Ser Pro Asp Gln Thr Arg Ser Glu Asp Pro Gln Asn Ser Ala
                165                 170                 175

Gly Ser Pro Pro Asp Ala Lys Ala Ser Asp Thr Pro Ser Thr Gly Ser
            180                 185                 190

Leu Gly Thr Asn Gln Asp Leu Ala Ser Gly Ser Glu Asn Asp Arg Asn
        195                 200                 205

Asp Ser Ala Ser Gln Pro Ser His Gln Ser Asp Ala Gly Lys Gln Gly
    210                 215                 220

Leu Gly Pro Pro Ser Thr Pro Ile Ala Val His Ala Ala Val Lys Ser
225                 230                 235                 240

Lys Ser Leu Gly Asp Ser Lys Asn Arg His Lys Lys Pro Lys Asp Pro
                245                 250                 255

Lys Pro Lys Val Lys Lys Leu Lys Tyr His Gln Tyr Ile Pro Pro Asp
            260                 265                 270

Gln Lys Ala Glu Lys Ser Pro Pro Pro Met Asp Ser Ala Tyr Ala Arg

275                 280                 285

Leu Leu Gln Gln Gln Gln Leu Phe Leu Gln Leu Gln Ile Leu Ser Gln
    290                 295                 300

Gln Gln Gln Gln Gln Gln His Arg Phe Ser Tyr Leu Gly Met His Gln
305                 310                 315                 320

Ala Gln Leu Lys Glu Pro Asn Glu Gln Met Val Arg Asn Pro Asn Ser
                325                 330                 335

Ser Ser Thr Pro Leu Ser Asn Thr Pro Leu Ser Pro Val Lys Asn Ser
            340                 345                 350

Phe Ser Gly Gln Thr Gly Val Ser Ser Phe Lys Pro Gly Pro Leu Pro
        355                 360                 365

Pro Asn Leu Asp Asp Leu Lys Val Ser Glu Leu Arg Gln Gln Leu Arg
    370                 375                 380

Ile Arg Gly Leu Pro Val Ser Gly Thr Lys Thr Ala Leu Met Asp Arg
385                 390                 395                 400

Leu Arg Pro Phe Gln Asp Cys Ser Gly Asn Pro Val Pro Asn Phe Gly
                405                 410                 415

Asp Ile Thr Thr Val Thr Phe Pro Val Thr Pro Asn Thr Leu Pro Asn
            420                 425                 430

Tyr Gln Ser Ser Ser Ser Thr Ser Ala Leu Ser Asn Gly Phe Tyr His
        435                 440                 445

Phe Gly Ser Thr Ser Ser Ser Pro Pro Ile Ser Pro Ala Ser Ser Asp
    450                 455                 460

Leu Ser Val Ala Gly Ser Leu Pro Asp Thr Phe Asn Asp Ala Ser Pro
465                 470                 475                 480

Ser Phe Gly Leu His Pro Ser Pro Val His Val Cys Thr Glu Glu Ser
                485                 490                 495

Leu Met Ser Ser Leu Asn Gly Gly Ser Val Pro Ser Glu Leu Asp Gly
            500                 505                 510

Leu Asp Ser Glu Lys Asp Lys Met Leu Val Glu Lys Gln Lys Val Ile
        515                 520                 525

Asn Glu Leu Thr Trp Lys Leu Gln Gln Glu Gln Arg Gln Val Glu Glu
    530                 535                 540

Leu Arg Met Gln Leu Gln Lys Gln Lys Arg Asn Asn Cys Ser Glu Lys
545                 550                 555                 560

Lys Pro Leu Pro Phe Leu Ala Ala Ser Ile Lys Gln Glu Glu Ala Val
                565                 570                 575

Ser Ser Cys Pro Phe Ala Ser Gln Val Pro Val Lys Arg Gln Ser Ser
            580                 585                 590

Ser Ser Glu Cys His Pro Pro Ala Cys Glu Ala Ala Gln Leu Gln Pro
        595                 600                 605

Leu Gly Asn Ala His Cys Val Glu Ser Ser Asp Gln Thr Asn Val Leu
    610                 615                 620

Ser Ser Thr Phe Leu Ser Pro Gln Cys Ser Pro Gln His Ser Pro Leu
625                 630                 635                 640

Gly Ala Val Lys Ser Pro Gln His Ile Ser Leu Pro Pro Ser Pro Asn
                645                 650                 655

Asn Pro His Phe Leu Pro Ser Ser Ser Gly Ala Gln Gly Glu Gly His
            660                 665                 670

Arg Val Ser Ser Pro Ile Ser Ser Gln Val Cys Thr Ala Gln Asn Ser
        675                 680                 685

Gly Ala His Asp Gly His Pro Pro Ser Phe Ser Pro His Ser Ser Ser
    690                 695                 700

Leu His Pro Pro Phe Ser Gly Ala Gln Ala Asp Ser Ser His Gly Ala
705                 710                 715                 720

Gly Gly Asn Pro Cys Pro Lys Ser Pro Cys Val Gln Gln Lys Met Ala
                725                 730                 735

Gly Leu His Ser Ser Asp Lys Val Gly Pro Lys Phe Ser Ile Pro Ser
            740                 745                 750

Pro Thr Phe Ser Lys Ser Ser Ser Ala Ile Ser Glu Val Thr Gln Pro
        755                 760                 765

Pro Ser Tyr Glu Asp Ala Val Lys Gln Gln Met Thr Arg Ser Gln Gln
    770                 775                 780

Met Asp Glu Leu Leu Asp Val Leu Ile Glu Ser Gly Glu Met Pro Ala
785                 790                 795                 800

Asp Ala Arg Glu Asp His Ser Cys Leu Gln Lys Val Pro Lys Ile Pro
                805                 810                 815

Arg Ser Ser Arg Ser Pro Thr Ala Val Leu Thr Lys Pro Ser Ala Ser
            820                 825                 830

Phe Glu Gln Ala Ser Ser Gly Ser Gln Ile Pro Phe Asp Pro Tyr Ala
        835                 840                 845

Thr Asp Ser Asp Glu His Leu Glu Val Leu Leu Asn Ser Gln Ser Pro
    850                 855                 860

Leu Gly Lys Met Ser Asp Val Thr Leu Leu Lys Ile Gly Ser Glu Glu
865                 870                 875                 880

Pro His Phe Asp Gly Ile Met Asp Gly Phe Ser Gly Lys Ala Ala Glu
                885                 890                 895

Asp Leu Phe Asn Ala His Glu Ile Leu Pro Gly Pro Leu Ser Pro Met
            900                 905                 910

Gln Thr Gln Phe Ser Pro Ser Ser Val Asp Ser Asn Gly Leu Gln Leu
        915                 920                 925

Ser Phe Thr Glu Ser Pro Trp Glu Thr Met Glu Trp Leu Asp Leu Thr
    930                 935                 940

Pro Pro Asn Ser Thr Pro Gly Phe Ser Ala Leu Thr Thr Ser Ser Pro
945                 950                 955                 960

Ser Ile Phe Asn Ile Asp Phe Leu Asp Val Thr Asp Leu Asn Leu Asn
                965                 970                 975

Ser Ser Met Asp Leu His Leu Gln Gln Trp
            980                 985

<210>  11
<211>  5127
<212>  DNA
<213>  Mus musculus

<400>  11
tggcagccta tgacatcagc caggaacgcc taggatgcgg ctgttcccgg ccaccctcag      60

aggaggaggg tcctgcctgc tgggagttaa ttagcctcgc gagcggcgag gggggaggcg     120

ccagttttct ggggacactg gcggccactg tgcgtcctcc tacccaaggg agctccccaa 180
gagttggatg aattctgggt tgttagctgc tgtcctctgg gctcccggga gccagtttct 240
ggtggaaagc ggggcgcctg gccaacgacc agcggcttgc tgagactcac catgacactc 300
ctggggtctg aacactcttt gctgattaga aggaagttcc gatcagtctt acagttacgg 360
cttcaacaga gaaggaccca ggagcagctg gctaaccaag gcttaatacc gccactgaaa 420
ggtccaactg aattccatga cccgagaaaa caattggata gtgccaagac tgaagattcc 480
ctgaggcgca agggcagaaa caggtccgac cgtgccagcc tggttactat gcacattctc 540
caagcctcca cggcagaaag gtccattcca actgctcaga tgaagctcaa aagagcccgc 600
cttgcagatg acctcaatga gaagatcgct ctccgcccag ggcccttgga actggtggag 660
aagaacattc tgccgatgga ttcttccgtg aaagaggcta taaaaggtac tgaggtgagc 720
ctctccaagg cagcagatgc attcgccttt gaggatgaca gcagtagaga tgggctctct 780
ccagatcagg ctaggagcga ggacccccag ggctctacag gatccacccc agacatcaaa 840
tccactgagg ctcctctgga cacaatccag gatctcactc ctggctcaga aagtgacaag 900
aatgatgcag cctcccagcc aggcaaccag tcagaccctg ggaagcaggt tctcggcccc 960
ctcagcaccc cgattcctgt gcacactgct gtaaagtcca agtctttggg tgacagtaag 1020
aaccgccaca aaaagcccaa agaccccaaa ccaaaggtga agaagctcaa ataccatcag 1080
tacatccccc cagaccagaa ggcagagaag tctcccccac ccatggactc tgcctatgcc 1140
cggctgctcc agcaacagca gctattcctg cagctacaga tcctcagcca gcagcagcaa 1200
cagcagcagc aacagcagca gcagcaacag cagcagcagc agcagcagca gcggttcagc 1260
taccctggga tgcaccaaac acacctcaaa gaaccaaatg aacagatggc cagaaatccg 1320
aatccttctt caacaccact gagcaatacc cctctatccc ctgtcaaaaa tagcatttct 1380
ggacaaactg gtgtttcttc tctcaaacca ggccccctcc cacccaacct ggatgatctc 1440
aaggtgtcag agttaagaca acagcttcga atccggggct tgccagtgtc aggcaccaag 1500
acagcgctgg tggaccggct tcgtcccttc caggattgtg ctggcaaccc tgtgcccaac 1560
tttggggaca tcacaactgt cacctttcct gtcacgccca acaccttgcc cagttatcag 1620
tcctccccga caggcttcta ccactttggc agcacaagct ccagcccacc catctccccc 1680
gcctcatctg acttgtccgc tgcagggtcc ctgccagaca ccttcaccga tgcgtcacct 1740
ggcttcggcc tgcacgcatc tccggtgccc gcctgcacgg acgagagtct gctgagcagc 1800
ctgaatgggg gctcgggccc ctccgagcct gatgggctag actctgagaa ggacaagatg 1860
ctggtggaga agcagaaagt gatcaaccag ctcacctgga agctgcggca agagcagcgg 1920
caggtggaag agctgagaat gcaactgcag aagcagaaga gcagctgcag cgaccagaag 1980
ccactgccct tcttggccac caccatcaaa caggaagatg tctccagctg ccccttcgca 2040

ccccagcagg cgtctgggaa gggacagggc cacagctctg acagtccccc tccggcttgt 2100

gagacggctc agctgctgcc tcactgtgtg gagtcctcag gtcaaaccca tgtactctcg 2160

tccacgtttc tcagccccca gtgctcccct cagcactcgc ccctgggggg cctgaagagc 2220

ccgcagcaca tcagcctgcc tccatcaccc aacaaccatt acttcctggc ttcctcttcg 2280

ggagctcaga gagagaacca tggggtctct tcacccagca gcagccaagg gtgcgcacag 2340

aactcagggg cacacgaagg ccattcttct agcttctctt ccccagcttc cagcctccat 2400

cagcctttct ctggcaccca agcagacagc agtcacagtg ctgggctcaa cccttgtccc 2460

aaaagcccaa gtattcatcc aaagatgact ggtttacaat cttctgacaa ggtggggcca 2520

acgttttcaa ttccatcccc aactttttct aagtcaagtt cagcagtttc agatatcacc 2580

cagcccccat cctatgaaga tgcagtgaag cagcaaatga ctcggagtca gcagatggac 2640

gaactcctgg atgtcctcat tgaaagtgga gaaatgccag ccgatgccag ggaagatcat 2700

tcatgtcttc agaaaattcc aaagatccct gggtcctcct gcagcccaac tgccatcccc 2760

ccgaagccct cggcttcctt tgagcaggca tcttcgggag gccagatggc cttcgatcac 2820

tacgccaacg acagtgacga acacctggaa gtcttattga attctcacag ccccatcgga 2880

aaggtgagcg atgttaccct cctcaaaatc ggaagcgagg agcctccttt tgacagcatc 2940

atggatggct tcccagggaa ggctgcggaa gatctcttca gtgctcacga gctcttgcct 3000

gggcccctct ccccgatgca tgcacagttg tcacctcctt ctgtggacag cagtggtctg 3060

cagctgagct tcacggaatc tccttgggaa acaatggaat ggctggacct cactccacct 3120

agttccacgc caggcttcag caaccttacc tccagtgggc ccagcatttt caacatcgat 3180

tttctggatg ttacagatct taatctgaat tcccctatgg atctccactt acagcagtgg 3240

taaacacccg aggtacaaga gctacgagag ctcagtggga attcaatgga ggaaagcacg 3300

ataccggaaa tgtgtgttcc aaaagatgaa gtggggaaaa tggggaggga aaaaaaaaaa 3360

cagcaacgga ggtttttgtg acaactaacc agaacaaaca gaagtcagct attaaaatat 3420

gtctaaatgt aatatctacc agcattcagt aactgttaat aacttcagtg atgcattcaa 3480

aaatgtgctt tgtcagaata agaatgccaa aaatgttttt tcgctgcctt atctcatacc 3540

agtttttttg gggttttttt tgtttgtttg ttttttggtt tttttttttt ttgtgttgtt 3600

atttggtttt ctttttgccc acagtttgtc tcaggcaata ctgggacata gactgacccc 3660

attagctttt gttatgaatt tactaaactt tctgtggaag gagaacagag cctctgccgc 3720

gggtgtgggg aagccatcct gtgcttgagg cagcacacgt gtgtccatca tcatcagtca 3780

gaagagcagg gcctgtctca cccaatcgag tccttaagac agaataatca gaatggtcag 3840

agggacagac caatcaattc ccaggaaagc aaaagtgact caatgtccct tgactcccaa 3900

atggtcccac tggactggtg atcactggtg acaactaact agctttgtcc agagaatcca 3960

cccagaacac ggtgcttttt agccagtagt ccacctctat gtgcatcagc aatgcatagc 4020

aggtgagaac ttgaatcaca gaaacttcat gccatggatg gagactcctg aggcgctcaa 4080

atactactac ctctagttcc aaagactaga gctagatgat cagaaaggca actggaggcc 4140

cagggagccg tactgggaca agttagaatt agagaacgat gtcatttaac attccgagaa 4200

agaaataacc atgaattgct attacaggag taacacacag ggccagcttc tttttcttct 4260

tttttatttt tcttttctta ttgtgagcag agggaattca cctcagttca tctttctctc 4320

agtacttttc tttcaagata tcaatccttt atgactcttt tgcttttaat tctctctctc 4380

tctctctctc tctctctttc tctcaaagga gaggtttcag ttctaacaag ctaccatact 4440

cctattaaag ccattttttt ttttagaata ttaaaagtcc aaactctctt gccaaactct 4500

ttcttcacat gcgcattggc tgaaaacaga atttacaaga atttctttag gaagaaactg 4560

gggatgtggc ccattggtca caaagttttt ttgtttgttt ttgtttttgt ttcaattctt 4620

gtttgattta tggacaatct ttggtttgta ttgctctgga gaaattggaa atcattgcag 4680

agtgaagata aatcagggca ccatgtatag tagagaatat ttcagtagtt ttccaaacga 4740

gaacacaatt gcacactgta aacaacagga gtgtgaagga ccacagtctt gaggagttct 4800

tgttgccctg cgtttggtga aggcgttggg gaccgaggaa gacaacatac agtttggcca 4860

aggctctcag aggcttgctg tggcgccaat tcaagtatta caatgttgca tgctgtagaa 4920

agtagctgtt gctgttgttt tgttttgttt taatttaagt caccaaggca ctgttttatt 4980

cttttgtaaa aaaaaaaaaa agttcactgt gcacttatag agaaaataat caacaatgtt 5040

gtgaattttt gagaagactt tttttttttt tttgataaac caaagattta gaaatcattc 5100

cattgtcaac ttgtaaaaaa aaaaaaa 5127

<210> 12
<211> 983
<212> PRT
<213> Mus musculus

<400> 12

Met Thr Leu Leu Gly Ser Glu His Ser Leu Leu Ile Arg Arg Lys Phe
1               5                   10                  15

Arg Ser Val Leu Gln Leu Arg Leu Gln Gln Arg Arg Thr Gln Glu Gln
            20                  25                  30

Leu Ala Asn Gln Gly Leu Ile Pro Pro Leu Lys Gly Pro Thr Glu Phe
        35                  40                  45

His Asp Pro Arg Lys Gln Leu Asp Ser Ala Lys Thr Glu Asp Ser Leu

50                  55                  60

Arg Arg Lys Gly Arg Asn Arg Ser Asp Arg Ala Ser Leu Val Thr Met
65                  70                  75                  80

His Ile Leu Gln Ala Ser Thr Ala Glu Arg Ser Ile Pro Thr Ala Gln
                85                  90                  95

Met Lys Leu Lys Arg Ala Arg Leu Ala Asp Asp Leu Asn Glu Lys Ile
            100                 105                 110

Ala Leu Arg Pro Gly Pro Leu Glu Leu Val Glu Lys Asn Ile Leu Pro
        115                 120                 125

Met Asp Ser Ser Val Lys Glu Ala Ile Lys Gly Thr Glu Val Ser Leu
    130                 135                 140

Ser Lys Ala Ala Asp Ala Phe Ala Phe Glu Asp Asp Ser Ser Arg Asp
145                 150                 155                 160

Gly Leu Ser Pro Asp Gln Ala Arg Ser Glu Asp Pro Gln Gly Ser Thr
                165                 170                 175

Gly Ser Thr Pro Asp Ile Lys Ser Thr Glu Ala Pro Leu Asp Thr Ile
            180                 185                 190

Gln Asp Leu Thr Pro Gly Ser Glu Ser Asp Lys Asn Asp Ala Ala Ser
        195                 200                 205

Gln Pro Gly Asn Gln Ser Asp Pro Gly Lys Gln Val Leu Gly Pro Leu
    210                 215                 220

Ser Thr Pro Ile Pro Val His Thr Ala Val Lys Ser Lys Ser Leu Gly
225                 230                 235                 240

Asp Ser Lys Asn Arg His Lys Lys Pro Lys Asp Pro Lys Pro Lys Val
                245                 250                 255

Lys Lys Leu Lys Tyr His Gln Tyr Ile Pro Pro Asp Gln Lys Ala Glu
            260                 265                 270

Lys Ser Pro Pro Pro Met Asp Ser Ala Tyr Ala Arg Leu Leu Gln Gln
        275                 280                 285

Gln Gln Leu Phe Leu Gln Leu Gln Ile Leu Ser Gln Gln Gln Gln Gln
    290                 295                 300

Gln Gln Gln Gln Gln Gln Gln Gln Gln Gln Gln Gln Gln Gln Gln Gln
305                 310                 315                 320

Arg Phe Ser Tyr Pro Gly Met His Gln Thr His Leu Lys Glu Pro Asn
                325                 330                 335

Glu Gln Met Ala Arg Asn Pro Asn Pro Ser Ser Thr Pro Leu Ser Asn
            340                 345                 350

Thr Pro Leu Ser Pro Val Lys Asn Ser Ile Ser Gly Gln Thr Gly Val
        355                 360                 365

Ser Ser Leu Lys Pro Gly Pro Leu Pro Pro Asn Leu Asp Asp Leu Lys
    370                 375                 380

Val Ser Glu Leu Arg Gln Gln Leu Arg Ile Arg Gly Leu Pro Val Ser
385                 390                 395                 400

Gly Thr Lys Thr Ala Leu Val Asp Arg Leu Arg Pro Phe Gln Asp Cys
                405                 410                 415

Ala Gly Asn Pro Val Pro Asn Phe Gly Asp Ile Thr Thr Val Thr Phe
            420                 425                 430

Pro Val Thr Pro Asn Thr Leu Pro Ser Tyr Gln Ser Ser Pro Thr Gly
        435                 440                 445

Phe Tyr His Phe Gly Ser Thr Ser Ser Ser Pro Pro Ile Ser Pro Ala
    450                 455                 460

Ser Ser Asp Leu Ser Ala Ala Gly Ser Leu Pro Asp Thr Phe Thr Asp
465                 470                 475                 480

Ala Ser Pro Gly Phe Gly Leu His Ala Ser Pro Val Pro Ala Cys Thr
                485                 490                 495

Asp Glu Ser Leu Leu Ser Ser Leu Asn Gly Gly Ser Gly Pro Ser Glu
            500                 505                 510

Pro Asp Gly Leu Asp Ser Glu Lys Asp Lys Met Leu Val Glu Lys Gln
        515                 520                 525

Lys Val Ile Asn Gln Leu Thr Trp Lys Leu Arg Gln Glu Gln Arg Gln
    530                 535                 540

Val Glu Glu Leu Arg Met Gln Leu Gln Lys Gln Lys Ser Ser Cys Ser
545                 550                 555                 560

Asp Gln Lys Pro Leu Pro Phe Leu Ala Thr Thr Ile Lys Gln Glu Asp
                565                 570                 575

Val Ser Ser Cys Pro Phe Ala Pro Gln Gln Ala Ser Gly Lys Gly Gln
            580                 585                 590

Gly His Ser Ser Asp Ser Pro Pro Pro Ala Cys Glu Thr Ala Gln Leu
        595                 600                 605

Leu Pro His Cys Val Glu Ser Ser Gly Gln Thr His Val Leu Ser Ser
    610                 615                 620

Thr Phe Leu Ser Pro Gln Cys Ser Pro Gln His Ser Pro Leu Gly Gly
625                 630                 635                 640

Leu Lys Ser Pro Gln His Ile Ser Leu Pro Pro Ser Pro Asn Asn His
                645                 650                 655

Tyr Phe Leu Ala Ser Ser Ser Gly Ala Gln Arg Glu Asn His Gly Val
            660                 665                 670

Ser Ser Pro Ser Ser Ser Gln Gly Cys Ala Gln Asn Ser Gly Ala His
        675                 680                 685

Glu Gly His Ser Ser Ser Phe Ser Ser Pro Ala Ser Ser Leu His Gln
    690                 695                 700

Pro Phe Ser Gly Thr Gln Ala Asp Ser Ser His Ser Ala Gly Leu Asn
705                 710                 715                 720

Pro Cys Pro Lys Ser Pro Ser Ile His Pro Lys Met Thr Gly Leu Gln
                725                 730                 735

Ser Ser Asp Lys Val Gly Pro Thr Phe Ser Ile Pro Ser Pro Thr Phe
            740                 745                 750

Ser Lys Ser Ser Ser Ala Val Ser Asp Ile Thr Gln Pro Pro Ser Tyr
        755                 760                 765

Glu Asp Ala Val Lys Gln Gln Met Thr Arg Ser Gln Gln Met Asp Glu
    770                 775                 780

Leu Leu Asp Val Leu Ile Glu Ser Gly Glu Met Pro Ala Asp Ala Arg
785                 790                 795                 800

Glu Asp His Ser Cys Leu Gln Lys Ile Pro Lys Ile Pro Gly Ser Ser
                805                 810                 815

Cys Ser Pro Thr Ala Ile Pro Pro Lys Pro Ser Ala Ser Phe Glu Gln
            820                 825                 830

Ala Ser Ser Gly Gly Gln Met Ala Phe Asp His Tyr Ala Asn Asp Ser
        835                 840                 845

Asp Glu His Leu Glu Val Leu Leu Asn Ser His Ser Pro Ile Gly Lys
    850                 855                 860

Val Ser Asp Val Thr Leu Leu Lys Ile Gly Ser Glu Glu Pro Pro Phe
865                 870                 875                 880

Asp Ser Ile Met Asp Gly Phe Pro Gly Lys Ala Ala Glu Asp Leu Phe
                885                 890                 895

Ser Ala His Glu Leu Leu Pro Gly Pro Leu Ser Pro Met His Ala Gln
            900                 905                 910

Leu Ser Pro Pro Ser Val Asp Ser Ser Gly Leu Gln Leu Ser Phe Thr
        915                 920                 925

Glu Ser Pro Trp Glu Thr Met Glu Trp Leu Asp Leu Thr Pro Pro Ser
    930                 935                 940

Ser Thr Pro Gly Phe Ser Asn Leu Thr Ser Ser Gly Pro Ser Ile Phe
945                 950                 955                 960

Asn Ile Asp Phe Leu Asp Val Thr Asp Leu Asn Leu Asn Ser Pro Met
                965                 970                 975

Asp Leu His Leu Gln Gln Trp
            980

## Description

### Technical Field

The present invention relates to a method for producing cardiac progenitor cells and cardiomyocytes from fibroblasts; and fibroblast-derived cardiac progenitor cells and fibroblast-derived cardiomyocytes, which are produced by the aforementioned method.

### Background Art

Heart disease has steadily increased with aging, and the incidence of heart failure in men aged 80 or over is high (14.7%). The heart is composed of cells such as cardiomyocytes and fibroblasts. Since cardiomyocytes having a beating function have almost no or completely no regeneration ability, the method for treating heart disease has been restricted so far.

To date, a method for directly producing cardiomyocyte-like cells from fibroblasts, without going through iPS cells, wherein the method comprises introduction of three cardiac reprogramming factors (Gata4, Mef2c and Tbx5; hereinafter referred to as "GMT"), has been found (Non Patent Literature 1). It was elucidated that, according to this method, the cardiac muscle can be directly produced from fibroblasts by the three factors GMT even in cultured cells and in the living body of a mouse (Patent Literature 1). Moreover, it has been reported that functionally immature cardiomyocyte-like cells can be produced from fibroblasts through cardiac progenitor cells, by using transcriptional factors (Mesp1 and Ets2) and a plurality of humoral factors (Non Patent Literature 2). Furthermore, a method of inducing cardiac progenitor cells or cardiomyocytes from pluripotent stem cells such as ES cells or iPS cells, using a humoral factor (Non Patent Literature 3), and a method of inducing cardiac progenitor cells from mouse ES cells, using a transcriptional factor, under the use of serum or under special culture conditions, have been reported (Non Patent Literature 4).

On the other hand, as described above, cardiomyocytes do not have proliferation ability. Accordingly, when cardiomyocytes are directly induced from fibroblasts or pluripotent stem cells, it is likely that the number of cells necessary for regenerative therapy could not be sufficiently obtained. Hence, a method for producing cardiomyocytes, comprising first producing cardiac progenitor cells having proliferation ability, and then producing cardiomyocytes from the cardiac progenitor cells, is useful.

However, a method for producing cardiac progenitor cells from fibroblasts, by using the aforementioned transcriptional factors (Mesp1 and Ets2) and a plurality of humoral factors, has been problematic in that the state of cardiac progenitor cells cannot be maintained, and that only functionally immature cardiomyocyte-like cells can be produced by the method.

### Citation List

### Patent Literature

Patent Literature 1: WO2011/139688

### Non Patent Literature

Non Patent Literature 1: Ieda, M., Fu, J.D., Delgado-Olguin, P., Vedantham, V., Hayashi, Y., Bruneau, B.G., and Srivastava, D. Direct Reprogramming of Fibroblasts into Functional Cardiomyocytes by Defined Factors. Cell 142: 375-386. 2010.
Non Patent Literature 2: Islas JF, Liu Y, Weng KC, et al. Transcription factors ETS2 and MESP1 transdifferentiate human dermal fibroblasts into cardiac progenitors. Proceedings of the National Academy of Sciences of the United States of America 2012; 109(32): 13016-21.
Non Patent Literature 3: Kattman SJ, Witty AD, Gagliardi M, et al. Stage-specific optimization of activin/nodal and BMP signaling promotes cardiac differentiation of mouse and human pluripotent stem cell lines. Cell stem cell 2011; 8(2): 228-40.
Non Patent Literature 4: van den Ameele J, Tiberi L, Bondue A, et al. Eomesodermin induces Mesp1 expression and cardiac differentiation from embryonic stem cells in the absence of Activin. EMBO reports 2012; 13(4): 355-62.

### Summary of Invention

### Technical Problem

Under the aforementioned circumstances, it has been desired to develop a method of inducing cardiac progenitor cells from fibroblasts, in which the induced cardiac progenitor cells are maintained for a certain period of time, and a method for producing functionally mature cardiomyocytes from the induced cardiac progenitor cells.

### Solution to Problem

As a result of intensive studies directed towards achieving the aforementioned objects, the present inventors have found that, when one factor (Tbx6) is introduced into fibroblasts, using a retroviral vector and retrovirus, cardiac progenitor cells, which express Mesp1 as a marker for the cardiac progenitor cells, are induced, and then, the induced cardiac progenitor cells proliferate to form a colony. In addition, the present inventors have also found that, in the induced cardiac progenitor cells, the expression of a plurality of cardiac progenitor cell gene markers is maintained even 1 month after the induction.

Moreover, the present inventors have found that, when three factors (Tbx6, SRF, and Myocd) are introduced into fibroblasts using a retroviral vector and retrovirus, beating cardiomyocytes are induced, and that the obtained cells express Nxk2.5 or troponin, which are markers for cardiomyocytes.

Furthermore, the present inventors have found that, when such three factors (Tbx6, SRF, and Myocd) are introduced into fibroblasts using a retroviral vector and retrovirus, the obtained cells express Myh11 as a marker for smooth muscle cells, and also that Pecam1 as a marker for vascular endothelial cells is expressed therein.

The present invention has been completed based on these findings.

Specifically, the present invention is as follows.
[1] A method for producing cardiac progenitor cells, comprising a step of introducing a Tbx6 gene into fibroblasts.
[2] A method for producing cardiomyocytes, comprising a step of introducing a Tbx6 gene, an SRF gene and a Myocardin gene into fibroblasts.
   The above-described fibroblasts are, for example, mouse cells or human cells.
[3] A cardiac progenitor cell derived from a fibroblast, comprising an exogenous Tbx6 gene.
[4] A cardiomyocyte derived from a fibroblast, comprising an exogenous Tbx6 gene, an exogenous SRF gene and an exogenous Myocardin gene.
[5] An inducer for inducing cardiac progenitor cells from fibroblasts, wherein the inducer comprises a Tbx6 gene.
[6] An inducer for inducing cardiomyocytes from fibroblasts, wherein the inducer comprises a Tbx6 gene, an SRF gene and a Myocd gene.
[7] An inducer for inducing smooth muscle cells from fibroblasts, wherein the inducer comprises a Tbx6 gene, an SRF gene and a Myocd gene.
[8] An inducer for inducing vascular endothelial cells from fibroblasts, wherein the inducer comprises a Tbx6 gene, an SRF gene and a Myocd gene.

### Advantageous Effects of Invention

According to the present invention, provided are a method for directly producing cardiac progenitor cells from fibroblasts, and a method for directly producing cardiomyocytes from fibroblasts. In addition, it is possible to provide cardiac progenitor cells and cardiomyocytes, which are produced by the method of the present invention. Since the cardiac progenitor cells induced from fibroblasts according to the present invention have maintained the expression of a plurality of cardiac progenitor cell genes, the present invention can provide a method for producing cardiac progenitor cells, which is more stable than conventional methods, and cardiac progenitor cells. Since cardiac progenitor cells have proliferation ability, the cardiac progenitor cells produced by the present invention can be preferably applied to medical use.

Moreover, the cardiomyocytes induced by the present invention have been confirmed to beat, and further, the expression of a cardiac muscle-specific gene or the expression of a structural protein has been confirmed in the present cardiomyocytes. Therefore, the method for producing cardiac muscle of the present invention can provide functionally mature cardiomyocytes.

### Brief Description of Drawings

Figure 1 is a schematic view showing introduction of a Tbx6 gene into fibroblasts to induce cardiac progenitor cells.
Figure 2 shows the immunostaining (MespCre-GFP) of Tbx6 gene-introduced cells with GFP (Mesp1 expression), and with DAPI (nuclear counterstaining), and the merged image of them (Merged).
Figure 3 is a view showing the mRNA expression levels of cardiac differentiation markers, Mesp1, T, KDR, Nkx2.5 and TnnT2, in cells induced by a Tbx6 gene, 1 month after the induction.
Figure 4 is a schematic view showing introduction of Tbx6, SRF, and Myocd (TSM) genes into fibroblasts, to induce beating cardiomyocytes.
Figure 5 is a view showing that cells, into which Tbx6, SRF and Myocd genes have been introduced, have a striated structure (bright field (BF)), and that the cells express cardiac troponin T.
Figure 6 is a view showing the mRNA expression levels of cardiac differentiation markers, Mesp1, T, KDR, Nkx2.5 and TnnT2, in the entire cells, into which Tbx6, SRF and Myocd genes have been introduced.
Figure 7 is a view showing the mRNA expression level of Myh11 as a marker for smooth muscle cells and the mRNA expression level of Pecam1 as a marker for vascular endothelial cells, in cells into which Tbx6, SRF and Myocd genes have been introduced.
Figure 8 is an immunostaining fluorescence microscopic image showing cells into which Tbx6, SRF and Myocd genes have been introduced, express a smooth muscle myosin heavy chain.

### Description of Embodiments

The present invention relates to a method for producing cardiac progenitor cells, comprising introducing a Tbx6 gene into fibroblasts, and fibroblast-derived cardiac progenitor cells, comprising an exogenous Tbx6 gene.

When a Tbx6 gene is introduced into fibroblasts by the use of retrovirus, cells, which express Mesp1 as a cardiac progenitor cell-specific marker, are induced. Even 30 days after introduction of Tbx6, such a cardiac progenitor cell-related gene is expressed, and the state of cardiac progenitor cells is maintained. A Tbx6 polypeptide is expressed in fibroblasts, and as a result, the Tbx6 gene-introduced fibroblasts are directly reprogrammed to differentiated cardiac progenitor cells, without going through stem cells or progenitor cells.

That is to say, the method for producing cardiac progenitor cells of the present invention comprises a step of introducing a Tbx6 gene into fibroblasts. According to the method for producing cardiac progenitor cells of the present invention, cardiac progenitor cells can be efficiently produced by introduction of only one factor. Moreover, since the expression of a marker gene in the cardiac progenitor cells is maintained even 1 month after the introduction of Tbx6, cardiac progenitor cells having proliferation ability can be stably produced according to the present invention.

Furthermore, the present invention relates to a method for producing cardiomyocytes, comprising introducing Tbx6, SRF and Myocardin (Myocd) genes into fibroblasts, and fibroblast-derived cardiomyocytes comprising an exogenous Tbx6 gene, an exogenous SRF gene and an exogenous Myocd gene.

When three factors (TSM), namely, Tbx6, and also, SRF and Myocd that are highly expressed in differentiated cardiomyocytes, are introduced into fibroblasts by the use of retrovirus, mature beating cardiomyocytes are induced. In addition, in the induced cardiomyocytes, the expression of cardiac muscle-specific genes such as Nkx2.5 or troponin, and the formation of a striated structure are confirmed. In fibroblasts, Tbx6, SRF and Myocd polypeptides are expressed, and as a result, the fibroblasts, into which the Tbx6, SRF and Myocd genes have been introduced, are directly reprogrammed to differentiated cardiomyocytes without going through stem cells or progenitor cells.

Further, when the Tbx6, SRF and Myocd genes are introduced into fibroblasts, not only the expression of cardiomyocytes-specific genes, but also the expression of cardiac progenitor cell genes (Mesp1, T, and KDR) is induced. Accordingly, since cell differentiation goes through cardiac progenitor cells according to the present invention, cardiomyocytes, smooth muscle cells, or vascular endothelial cells can be produced.

That is to say, the method for producing cardiomyocytes of the present invention comprises a step of introducing a Tbx6 gene, an SRF gene and a Myocd gene into fibroblasts. According to the method for producing cardiomyocytes of the present invention, functionally mature cardiomyocytes can be produced.

Hence, the present invention provides fibroblast-derived cardiac progenitor cells, into which a Tbx6 gene has been introduced, or fibroblast-derived cardiomyocytes, into which a Tbx6 gene, an SRF gene and a Myocd gene have been introduced. In addition, the present invention provides an inducer for inducing cardiac progenitor cells, which comprises a Tbx6 gene, or an inducer for inducing cardiomyocytes, which comprises a Tbx6 gene, an SRF gene, and a Myocd gene. Moreover, since not only cardiomyocytes, but also smooth muscle cells or endothelial cells are induced by introducing a Tbx6 gene, an SRF gene and a Myocd gene into fibroblasts, according to the present invention, an inducer for inducing smooth muscle cells, or an inducer for inducing endothelial cells, each of which comprises a Tbx6 gene, an SRF gene, and a Myocd gene, is provided.

### [Direct reprogramming]

In the present invention, fibroblasts, into which a Tbx6 gene has been introduced, or fibroblasts, into which a Tbx6 gene, an SRF gene and a Myocd gene have been introduced, are directly reprogrammed to differentiated cardiac progenitor cells or differentiated cardiomyocytes, without going through stem cells or progenitor cells.

### [Introduction of reprogramming factors]

In one aspect of the present invention, a Tbx6 gene, or a set of a Tbx6 gene, an SRF gene and a Myocd gene can be introduced into fibroblasts *in vitro.* In addition, the fibroblasts are induced to differentiate into cardiac progenitor cells or cardiomyocytes *in vitro.* The induced cardiac progenitor cells or the induced cardiomyocytes can be introduced into an individual body.

In another aspect of the present invention, a Tbx6 gene, or a set of a Tbx6 gene, an SRF gene and a Myocd gene can be introduced *in vivo* into fibroblasts, for example, into the diseased cardiac tissues of an individual body. In addition, the fibroblasts are induced to differentiate into cardiac progenitor cells or cardiomyocytes *in vivo.*

In another aspect of the present invention, a Tbx6 gene, or a set of a Tbx6 gene, an SRF gene and a Myocd gene can be introduced into fibroblasts *in vitro.* In addition, the fibroblasts are introduced into an individual body, and are then induced to differentiate into cardiac progenitor cells or cardiomyocytes *in vivo.*

Introduction of a reprogramming factor gene (a Tbx6 gene, an SRF gene or a Myocd gene) into fibroblasts can be carried out by introducing a nucleic acid comprising a nucleotide sequence encoding Tbx6, a nucleic acid comprising a nucleotide sequence encoding SRF, or a nucleic acid comprising a nucleotide sequence encoding Myocd, into fibroblasts. The thus gene-introduced fibroblasts are induced to differentiate into cardiac progenitor cells or cardiomyocytes by expressing Tbx6, SRF or Myocd. Accordingly, the step of introducing a Tbx6 gene into fibroblasts may comprise introducing a Tbx6 polypeptide into fibroblasts. On the other hand, the step of introducing a Tbx6 gene, an SRF gene and a Myocd gene into fibroblasts may comprise introducing a Tbx6 polypeptide, an SRF polypeptide and a Myocd polypeptide into fibroblasts.

Moreover, the method for producing cardiac progenitor cells or cardiomyocytes of the present invention may comprise a step of transforming fibroblasts using a Tbx6 gene, or a step of transforming fibroblasts using a Tbx6 gene, an SRF gene and a Myocd gene. Furthermore, the method for producing cardiac progenitor cells or cardiomyocytes of the present invention may comprise a step of expressing a Tbx6 gene in fibroblasts, or a step of expressing a Tbx6 gene, an SRF gene and a Myocd gene in fibroblasts.

Fibroblasts, into which a Tbx6 gene has been introduced, or fibroblasts, into which a Tbx6 gene, an SRF gene and a Myocd gene have been introduced, are induced to differentiate into cardiac progenitor cells or cardiomyocytes within a certain period of time, for example, within 7 to 14 days, and preferably within 7 days. For example, when a Tbx6 gene, or a set of a Tbx6 gene, an SRF gene and a Myocd gene is introduced into a fibroblast population, at least 10%, at least 15%, at least 20%, at least 30%, at least 50%, at least 70%, or at least 90% of the population is reprogrammed to cardiac progenitor cells or cardiomyocytes, for example, within a period of 7 to 14 days, and preferably within a period of 7 days.

In the method for producing cardiac progenitor cells or cardiomyocytes of the present invention, a certain period of time (for example, 7 to 14 days, and preferably 7 days) after the step of introducing a Tbx6 gene, or a set of a Tbx6 gene, an SRF gene and a Myocd gene into fibroblasts a step of sorting a population of the fibroblasts is carried out, so that the ratio of the cardiac progenitor cells or the cardiomyocytes can be enriched. Such a sorting step is performed regarding the positive expression of a fibroblast-specific marker such as vimentin, poly-1-4-hydroxylase, a fibroblast-specific protein, a fibroblast surface antigen, or type 1 collagen, so that remaining fibroblasts can be removed if they remain. In addition, a sorting step is performed regarding the expression of a marker specific to cardiac progenitor cells or cardiomyocytes, so that the ratio of such cells can be enriched.

Moreover, when the method for producing cardiac progenitor cells or cardiomyocytes of the present invention comprises a step of introducing a nucleic acid comprising a nucleotide sequence encoding a detectable marker into fibroblasts, it can give a means for the sorting step, or a means for confirming induction of differentiation into cardiac progenitor cells or cardiomyocytes. Such a nucleotide sequence encoding a detectable marker is operably linked to a cardiac progenitor cell-specific promoter or a cardiomyocyte-specific promoter, or is linked to a nucleotide sequence encoding a cardiac progenitor cell-specific marker or a nucleotide sequence encoding a cardiomyocyte-specific marker. Examples of the detectable marker include: polypeptides directly generating detectable signals, for example, fluorescent proteins such as GFP, YEP, or BFP; and enzymes generating detectable signals when they act on a substrate, such as luciferase or alkaline phosphatase. Examples of the cardiac progenitor cell-specific promoter include promoters of Mesp1, T, or Flk1 (KDR). Examples of the promoter specific to cardiomyocytes include an α-myosin heavy chain promoter and a cTnT promoter. The expression of a detectable marker enables detection of cardiac progenitor cells or cardiomyocytes, and as a result, it can give a means for confirming induction of differentiation into cardiac progenitor cells or cardiomyocytes, or sorting the induced cardiac progenitor cells or the induced cardiomyocytes.

In the present description, the phrase "operably linking" is used to mean functional linking between nucleic acids, which gives a desired function such as transcription or translation. For example, this linking includes functional linking between a nucleic acid expression control sequence such as a promoter or a signal sequence, and a second polynucleotide. The expression control sequence has an influence on the transcription and/or translation of the second polynucleotide.

### [Fibroblasts]

In the present invention, as fibroblasts, mammalian fibroblasts, such as human fibroblasts, or the fibroblasts of mammals other than a human, such as a mouse, a rat, a swine, a monkey, a horse, a bovine, sheep, a goat or a dog, can be used. The fibroblasts are preferably human fibroblasts. In another embodiment, the fibroblasts are mouse fibroblasts. The fibroblasts may be fibroblasts obtained from mammals, or may also be progenies isolated from the fibroblasts obtained from mammals, or may further be the sub-cultured cells thereof. As such fibroblasts, for example, embryonic fibroblasts, tail tip-derived fibroblasts, cardiac fibroblasts, foreskin fibroblasts, skin fibroblasts, lung fibroblasts, etc. can be used.

A medium used in the culture of fibroblasts, such as MEM, DMEM, or IMDM medium, can be appropriately selected or prepared by a person skilled in the art. The fibroblasts can be cultured in the presence or absence of serum. The culture is not particularly limited, as long as it is carried out under conditions suitable for the culture of fibroblasts. In general, fibroblasts are cultured in a temperature range of 25°C to 37°C under conditions of 5% CO₂.

### [Reprogramming factor]

In the method for producing cardiac progenitor cells or cardiomyocytes of the present invention, one or more nucleic acids comprising nucleotide sequences encoding one or more reprogramming factors can be introduced into fibroblasts. Otherwise, in the method for producing cardiac progenitor cells or cardiomyocytes of the present invention, one or more reprogramming factor polypeptides themselves can be introduced into fibroblasts. In the present invention, when cardiac progenitor cells are to be produced, the reprogramming factor is Tbx6. When cardiomyocytes are to be produced, the reprogramming factors are Tbx6, SRF, and Myocd (TSM). Tbx6, SRF, and Myocd can be introduced, at once or successively, into cells. The phrase "introduction into cells at once" means that a plurality of reprogramming factors are introduced into cells by a single cell introduction step. On the other hand, the phrase "introduction into cells successively" means that a plurality of reprogramming factors are introduced into cells by a plurality of cell introduction steps on the same day or on the different days. From the viewpoint of cell introduction efficiency, it is preferable to introduce a plurality of reprogramming factors into cells at once. In addition, the amino acid sequences of the reprogramming factors of the present invention, and nucleotide sequences encoding the amino acid sequences are known in the present technical field.

### [Tbx6]

A Tbx6 polypeptide (T-box transcriptional factor 6) is a transcriptional factor that binds to T box in the promoter region of a certain gene and recognizes it. The amino acid sequences of Tbx6 polypeptides derived from various species, and nucleotide sequences encoding the Tbx6 polypeptides derived from various species have been known. For example, Genbank Accession Nos. NM_004608.3 (human, nucleotide sequence, SEQ ID NO: 1, CDS 61..1371), NP_004599.2 (human, amino acid sequence, SEQ ID NO: 2), NM_011538.2 (mouse, nucleotide sequence, SEQ ID NO: 3, CDS 25..1335), NP_035668.2 (mouse, amino acid sequence, SEQ ID NO: 4), and the like may be referred to.

Moreover, in one aspect of the present invention, the Tbx6 polypeptide includes a polypeptide, which has an amino acid sequence having a sequence identity of at least 80%, 85%, 90%, 91%, 92%, 93%, 94%, 95%, 96%, 97%, 98%, 99% or 100% to the amino acid sequence shown in SEQ ID NO: 2 or SEQ ID NO: 4, and has a function of inducing fibroblasts to differentiate into cardiac progenitor cells, when it is introduced into the fibroblasts.

Furthermore, in one aspect of the present invention, the Tbx6 polypeptide includes a polypeptide, which has an amino acid sequence comprising a deletion, substitution, insertion or addition of 1 to 50, preferably 1 to 40, more preferably 1 to 20, and further preferably 1 to 10 (for example, 1, 2, 3, 4, 5, 6, 7, 8, 9 or 10) amino acids, or a combination thereof, with respect to the amino acid sequence shown in SEQ ID NO: 2 or SEQ ID NO: 4, and has a function of inducing fibroblasts to differentiate into cardiac progenitor cells, when it is introduced into the fibroblasts.

Further, in one aspect of the present invention, the Tbx6 polypeptide includes a polypeptide, which has an amino acid sequence encoded by a nucleotide sequence having a sequence identity of at least 80%, 85%, 90%, 91%, 92%, 93%, 94%, 95%, 96%, 97%, 98%, 99% or 100% to the nucleotide sequence shown in SEQ ID NO: 1 or SEQ ID NO: 3, and has a function of inducing fibroblasts to differentiate into cardiac progenitor cells, when it is introduced into the fibroblasts.

Still further, in one aspect of the present invention, the Tbx6 gene (nucleic acid) includes a nucleic acid, which has a nucleotide sequence having a sequence identity of at least 80%, 85%, 90%, 91%, 92%, 93%, 94%, 95%, 96%, 97%, 98%, 99% or 100% to the nucleotide sequence shown in SEQ ID NO: 1 or SEQ ID NO: 3, and has a function of inducing fibroblasts to differentiate into cardiac progenitor cells, when the polypeptide encoded by the nucleic acid is introduced into the fibroblasts.

### [SRF]

The amino acid sequences of SRF polypeptides derived from various species, and nucleotide sequences encoding the SRF polypeptides derived from various species have been known. For example, Genbank Accession Nos. NM_003131.3 (human, nucleotide sequence, SEQ ID NO: 5, CDS 363..1889), NP_003122.1 (human, amino acid sequence, SEQ ID NO: 6), NM_020493.2 (mouse, nucleotide sequence, SEQ ID NO: 7, CDS 335..1849), NP_065239.1 (mouse, amino acid sequence, SEQ ID NO: 8), and the like may be referred to.

Moreover, in one aspect of the present invention, the SRF polypeptide includes a polypeptide, which has an amino acid sequence having a sequence identity of at least 80%, 85%, 90%, 91%, 92%, 93%, 94%, 95%, 96%, 97%, 98%, 99% or 100% to the amino acid sequence shown in SEQ ID NO: 6 or SEQ ID NO: 8, and has a function of inducing fibroblasts to differentiate into cardiomyocytes, when it is introduced, together with Tbx6 and Myocardin, into the fibroblasts.

Furthermore, in one aspect of the present invention, the SRF polypeptide includes a polypeptide, which has an amino acid sequence comprising a deletion, substitution, insertion or addition of 1 to 60, preferably 1 to 50, more preferably 1 to 25, and further preferably 1 to 13 (for example, 1, 2, 3, 4, 5, 6, 7, 8, 9, 10, 11, 12 or 13) amino acids, or a combination thereof, with respect to the amino acid sequence shown in SEQ ID NO: 6 or SEQ ID NO: 8, and has a function of inducing fibroblasts to differentiate into cardiomyocytes, when it is introduced, together with Tbx6 and Myocardin, into the fibroblasts.

Further, in one aspect of the present invention, the SRF polypeptide includes a polypeptide, which has an amino acid sequence encoded by a nucleotide sequence having a sequence identity of at least 80%, 85%, 90%, 91%, 92%, 93%, 94%, 95%, 96%, 97%, 98%, 99% or 100% to the nucleotide sequence shown in SEQ ID NO: 5 or SEQ ID NO: 7, and has a function of inducing fibroblasts to differentiate into cardiomyocytes, when it is introduced, together with Tbx6 and Myocardin, into the fibroblasts.

Still further, in one aspect of the present invention, the SRF nucleic acid includes a nucleic acid, which has a nucleotide sequence having a sequence identity of at least 80%, 85%, 90%, 91%, 92%, 93%, 94%, 95%, 96%, 97%, 98%, 99% or 100% to the nucleotide sequence shown in SEQ ID NO: 5 or SEQ ID NO: 7, and has a function of inducing fibroblasts to differentiate into cardiomyocytes, when it is introduced, together with Tbx6 and Myocardin, into the fibroblasts.

### [Myocardin] (Myocd)

The amino acid sequences of Myocardin polypeptides derived from various species, and nucleotide sequences encoding the Myocardin polypeptides derived from various species have been known. For example, Genbank Accession Nos. NM_001146312.2 (human, nucleotide sequence, SEQ ID NO: 9, CDS 300..3260), NP_001139784.1 (human, amino acid sequence, SEQ ID NO: 10), NM_145136.4 (mouse, nucleotide sequence, SEQ ID NO: 11, CDS 292..3243), NP_660118.3 (mouse, amino acid sequence, SEQ ID NO: 12), and the like may be referred to.

Moreover, in one aspect of the present invention, the Myocardin polypeptide includes a polypeptide, which has an amino acid sequence having a sequence identity of at least 80%, 85%, 90%, 91%, 92%, 93%, 94%, 95%, 96%, 97%, 98%, 99% or 100% to the amino acid sequence shown in SEQ ID NO: 10 or SEQ ID NO: 12, and has a function of inducing fibroblasts to differentiate into cardiomyocytes, when it is introduced, together with Tbx6 and SRF, into the fibroblasts.

Furthermore, in one aspect of the present invention, the Myocardin polypeptide includes a polypeptide, which has an amino acid sequence comprising a deletion, substitution, insertion or addition of 1 to 100, preferably 1 to 50, more preferably 1 to 30, and further preferably 1 to 20 (for example, 1, 2, 3, 4, 5, 6, 7, 8, 9, 10, 11, 12, 13, 14, 15, 16, 17, 18, 19 or 20) amino acids, or a combination thereof, with respect to the amino acid sequence shown in SEQ ID NO: 10 or SEQ ID NO: 12, and has a function of inducing fibroblasts to differentiate into cardiomyocytes, when it is introduced, together with Tbx6 and SRF, into the fibroblasts.

Further, in one aspect of the present invention, the Myocardin polypeptide includes a polypeptide, which has an amino acid sequence encoded by a nucleotide sequence having a sequence identity of at least 80%, 85%, 90%, 91%, 92%, 93%, 94%, 95%, 96%, 97%, 98%, 99% or 100% to the nucleotide sequence shown in SEQ ID NO: 9 or SEQ ID NO: 11, and has a function of inducing fibroblasts to differentiate into cardiomyocytes, when it is introduced, together with Tbx6 and SRF, into the fibroblasts.

Still further, in one aspect of the present invention, the Myocardin gene (nucleic acid) includes a nucleic acid, which has a nucleotide sequence having a sequence identity of at least 80%, 85%, 90%, 91%, 92%, 93%, 94%, 95%, 96%, 97%, 98%, 99% or 100% to the nucleotide sequence shown in SEQ ID NO: 9 or SEQ ID NO: 11, and has a function of inducing fibroblasts to differentiate into cardiomyocytes, when it is introduced, together with Tbx6 and SRF, into the fibroblasts.

To date, it has been reported that functionally immature cardiomyocyte-like cells can be produced from fibroblasts, through cardiac progenitor cells, by using transcriptional factors (Mesp1 and Ets2) and a plurality of humoral factors (Non Patent Literature 2). In the present invention, such transcriptional factors do not need to be introduced into fibroblasts, and also, such transcriptional factors do not need to be combined with humoral factors.

### [Cardiac progenitor cells]

In the present invention, the "cardiac progenitor cells" are characterized in that the cells express markers specific to the cardiac progenitor cells. The markers specific to cardiac progenitor cells are factors that are specifically expressed in cardiac progenitor cells (cardiac progenitor cell-related factors). Examples of the marker specific to cardiac progenitor cells include T, Mesp1, Flk1 (KDR), Pdgfrα, and Isl1. Cardiac progenitor cells express at least one, more preferably at least two, and further preferably at least three of such cardiac progenitor cell-specific markers. Cardiac progenitor cells are preferably cells, which express T, Mesp1, and Flk1 (KDR). The expression of a marker can be confirmed at a gene level or a protein level.

In addition, in the present invention, since cardiac progenitor cells are induced from fibroblasts, the cardiac progenitor cells may also be referred to as "induced cardiac progenitor cells."

### [Cardiomyocytes]

In the present invention, the "cardiomyocytes" are characterized in that the cells express markers specific to the cardiomyocytes. The markers specific to cardiomyocytes are factors that are specifically expressed in cardiomyocytes (myocardial cell-related factors). Examples of the marker specific to cardiomyocytes include cardiac troponin (cTnT), Nkx2.5, and Actn2. Cardiomyocytes express at least one, more preferably at least two, and further preferably at least three of such cardiomyocyte-specific markers. Cardiomyocytes are preferably cells, which express cTnT and Nkx2.5.

Moreover, in the present invention, the "cardiomyocytes" may be characterized in that the cells beat. Furthermore, in the present invention, the "cardiomyocytes" may also be characterized in that the cells form a striated structure.

Further, in the present invention, since cardiomyocytes are induced from fibroblasts, the cardiomyocytes may also be referred to as "induced cardiomyocytes."

The expression of various markers specific to cardiac progenitor cells or cardiomyocytes can be detected by biochemical or immunochemical approaches (for example, an enzyme-linked immunosorbent assay, an immunohistochemical assay, etc.). Alternatively, the expression of such markers can also be detected by measuring the expression of nucleic acids encoding various markers specific to cardiac progenitor cells or cardiomyocytes. The expression of such nucleic acids encoding various markers specific to cardiac progenitor cells or cardiomyocytes can be confirmed by molecular biological approaches such as RT-PCR or hybridization. Primers or probes used in these approaches can be appropriately designed and produced by a person skilled in the art, using information available from database such as Genbank.

The beating of cardiomyocytes can be confirmed by visual observation or by observing the bright field image thereof. In addition, it is also possible to confirm spontaneous contraction by standard electrophysiological methods such as a patch clamp method.

Moreover, the formation of a striated structure by cardiomyocytes can be confirmed by visual observation or by observing the bright field image thereof. Furthermore, it can also be confirmed by performing immunostaining on proteins contributing to a cardiac muscle structure, such as troponin.

### [Introduction of exogenous reprogramming factor polypeptides into fibroblasts]

In the present description, the term "exogenous" means a nucleic acid or a polypeptide to be introduced into certain cells (for example, by electroporation, infection, lipofection, microinjection, or any other methods of introducing a nucleic acid into cells).

In one aspect of the present invention, the exogenous reprogramming factors can also be introduced into fibroblasts by allowing the polypeptides of Tbx6, SRF and/or Myocd to come into contact with the fibroblasts. The polypeptides of Tbx6, SRF, and Myocd can be each produced by a genetically engineering method or a molecular biological method, based on information regarding amino acid sequences and nucleotide sequences stored in known database.

### [Introduction of exogenous reprogramming factor genes into fibroblasts]

Introduction of the exogenous reprogramming factor (Tbx6, SRF, and Myocd) genes, namely, introduction of polynucleotides encoding these polypeptides or polynucleotides having nucleotide sequences complementary to the nucleotide sequences thereof, can be carried out by known transformation methods, such as viral infection using, for example, viral vectors such as a retroviral vector or an adenoviral vector, a lipofection method, an electroporation method, a microinjection method, or a calcium phosphate method.

In another aspect of the present invention, introduction of exogenous reprogramming factor polypeptides (Tbx6, or a set of Tbx6, SRF and Myocd) into fibroblasts is achieved by introducing exogenous nucleic acids comprising nucleotide sequences encoding the reprogramming factor polypeptides into fibroblasts. The species as origins of the exogenous reprogramming factors are preferably identical to the species as origins of the fibroblasts, such as, for example, a human and a human, or a mouse and a mouse.

In the present invention, an exogenous nucleic acid comprising a nucleotide sequence encoding an exogenous reprogramming factor polypeptide can be in the form of a recombinant expression vector comprising an expression cassette. In such a case, examples of a suitable vector include: recombinant retrovirus, lentivirus, and adenovirus; and a retrovirus expression vector, a lentivirus expression vector, a nucleic acid expression vector, and a plasmid expression vector. In another aspect of the present invention, an exogenous nucleic acid is incorporated into the genome of fibroblasts and the progenies thereof.

In an aspect of the present invention, fibroblasts are transformed with different expression constructs (expression vectors) each comprising a nucleotide sequence encoding Tbx6, SRF, or Myocd. In another aspect of the present invention, the expression construct may comprise nucleotide sequences encoding two or more of Tbx6, SRF, and Myocd. In an aspect of the present invention, the expression construct comprises nucleotide sequences encoding Tbx6, SRF, and Myocd.

In the present invention, an exogenous nucleic acid comprising a nucleotide sequence encoding the Tbx6 polypeptide, or an exogenous nucleic acid(s) comprising a nucleotide sequence(s) encoding one or more of Tbx6, SRF and Myocd polypeptides, is (are) introduced *in vitro* into a single fibroblast or a population of fibroblasts, or is (are) introduced *in vivo* in a single fibroblast or a population of fibroblasts.

In another aspect of the present invention, a nucleic acid(s) comprising a nucleotide sequence(s) encoding the Tbx6 polypeptide, or one or more of the Tbx6, SRF and Myocd polypeptides, may be an expression construct(s) capable of production of the reprogramming factor polypeptide(s) in fibroblasts. In another aspect of the present invention, examples of the expression construct include viral constructs such as a recombinant adeno-associated viral construct (see, for example, U.S. Patent No. 7,078,387), a recombinant adenoviral construct, and a recombinant lentiviral construct.

Examples of a suitable expression vector include viral vectors (e.g., vaccinia virus-based viral vectors; polio virus; adenovirus (see, for example, Li et al., Invest Opthalmol Vis Sci 35: 2543 2549, 1994; Borras et al., Gene Ther 6: 515 524, 1999; Li and Davidson, PNAS 92: 7700 7704, 1995; Sakamoto et al., H Gene Ther 5: 1088 1097, 1999; International Publication WO No. 94/12649; International Publication WO No. 93/03769; International Publication WO No. 93/19191; International Publication WO No. 94/28938; International Publication WO No. 95/1 1984; and International Publication WO No. 95/00655); adeno-associated virus (see, for example, Ali et al., Hum Gene Ther 9: 81 86, 1998, Flannery et al., PNAS 94: 6916 6921, 1997; Bennett et al., Invest Opthalmol Vis Sci 38: 2857-2863, 1997; Jomary et al., Gene Ther4: 683-690, 1997, Rolling et al., Hum Gene Ther 10: 641 648, 1999; Ali et al., Hum Mol Genet 5: 591-594, 1996; Srivastava, International Publication WO No. 93/09239, Samulski et al., J. Vir. (1989) 63: 3822 3828; Mendelson et al., Virol. (1988) 166: 154 165; and Flotte et al., PNAS (1993) 90: 10613-10617); SV40; herpes simplex virus; human immunodeficiency virus (see, for example, Miyoshi et al., PNAS94: 10319-23, 1997; Takahashi et al., J Virol 73: 7812 7816, 1999); retroviral vectors (e.g., vectors derived from murine leukemia virus, spleen necrosis virus, and retrovirus, for example, Rous sarcomere virus, Harvey sarcomere virus, avian leukemia virus, lentivirus, human immunodeficiency virus, myeloproliferative sarcomere virus, and breast cancer virus), but the examples of a suitable expression vector are not limited thereto.

A large number of suitable expression vectors have been known in the present technical field, and many expression vectors are commercially available. The below-mentioned vectors are presented for illustrative purposes, and for eukaryotic host cells, pXT1, pSG5 (Stratagene), pSVK3, pBPV, pMSG, and pSVLSV40 (Pharmacia) are used. However, any other vectors can also be used as long as they are compatible with host cells.

Depending on the used host/vector system, any of many suitable transcriptional and translational regulatory elements, such as constitutive and inducible promoters, transcriptional enhancer elements, and transcriptional terminators, may be used in an expression vector (see, for example, BITTER et al. (1987) METHODS IN ENZYMOLOGY, 153: 516-544).

In another aspect of the present invention, nucleotide sequences encoding reprogramming factors (e.g., Tbx6 CDR sequence, SRF CDR sequence, and Myocd CDR sequence) may be operably linked to regulatory elements, for example, to transcriptional regulatory elements, such as promoters. The transcriptional regulatory elements function in eukaryotic cells, such as in mammalian cells. Suitable transcriptional regulatory elements include promoters and enhancers. In another aspect of the present invention, promoters are constitutively active. In another embodiment, promoters are inducible.

Non-restrictive examples of a suitable eukaryotic promoter (a promoter functioning in eukaryotic cells) include CMV immediate early, HSV thymidine kinase, early and late SV40, retrovirus-derived long terminal repeat (LTR), and mouse metallothionein-I.

In another aspect of the present invention, a nucleotide sequence encoding a reprogramming factor is operably linked to heart-specific transcriptional regulatory elements (TRE), and in such a case, TRE may include promoters and enhancers. Suitable TRE includes TRE derived from the below-mentioned genes, namely, from myosin light chain-2, α-myosin heavy chain, AE3, cardiac troponin C, and cardiac actin, but the examples of the TRE are not limited thereto (Franz et al. (1997) Cardiovasc. Res. 35: 560-566; Robbins et al. (1995) Ann. N. Y. Acad. Sci. 752: 492-505; Linn et al. (1995) Circ. Res. 76: 584-591;Parmacek et al. (1994) Mol. Cell. Biol. 14: 1870-1885; Hunter et al. (1993) Hypertension 22:608-617; and Sartorelli et al. (1992) Proc. Natl. Acad. Sci. USA 89: 4047-4051.).

Selection of a suitable vector and a suitable promoter is well known in the present technical field. The expression vector may comprise a ribosome binding site for initiation of translation and initiation of transcription. The expression vector may comprise a suitable sequence for amplifying the expression.

Examples of a suitable mammalian expression vector (an expression vector suitable for use in mammalian host cells) include recombinant virus, nucleic acid vectors, such as a plasmid, a bacterial artificial chromosome, a yeast artificial chromosome, a human artificial chromosome, cDNA, cRNA, and a polymerase chain reaction (PCR) product expression cassette, but the examples of a suitable mammalian expression vector are not limited thereto. Examples of a suitable promoter for driving the expression of nucleotide sequences encoding Tbx6, SRF, and Myocd include retrovirus long terminal repeat (LTR) element; constitutive promoters such as CMV, HSV1-TK, SV40, EF-1α, or β-actin; and phosphoglycerol kinase (PGK), and inducible promoters, such as those containing a Tet-operator element, but the examples of the suitable promoter are not limited thereto. In some cases, such a mammalian expression vector may encode a marker gene that facilitates discrimination or selection of transfected or infected cells, as well as exogenous Tbx6, SRF and Myocd polypeptides. Examples of the marker gene include: genes encoding fluorescent proteins such as an enhanced green fluorescent protein, Ds-Red (DsRed: Discosomasp. red fluorescent protein (RFP); Bevis and Glick (2002) Nat. Biotechnol. 20: 83), a yellow fluorescent protein, and a cyan fluorescent protein; and genes encoding proteins that impart resistance to selective agents, such as a neomycin resistance gene, a puromycin resistance gene, and a blasticidin resistance gene, but the examples of the marker gene are not limited thereto.

Examples of a suitable viral vector include: a retrovirus-based viral vector (including lentivirus); adenovirus; and adeno-associated virus, but the examples are not limited thereto. A suitable retrovirus-based vector is Moloney murine leukemia virus (MMLV)-based vector, but other recombinant retroviruses may also be used. Examples of such other recombinant retroviruses include avian leukemia virus, bovine leukemia virus, murine leukemia virus (MLV), mink cell focus inducing virus, murine sarcomere virus, reticuloendotheliosis virus, gibbon ape leukemia virus, Mason Pfizer monkey virus, and Rous sarcomere virus. For instance, U.S. Patent No. 6,333,195 can be referred to.

In another case, the retrovirus-based vector may be a lentivirus-based vector (e.g., human immunodeficiency virus-1 (HIV-1); simian immunodeficiency virus (SIV); or ferine immunodeficiency virus (FIV)), and for instance, Johnston et al. (1999), Journal of Virology, 73(6): 4991-5000 (FIV); Negre D et al. (2002), Current Topics in Microbiology and Immunology, 261: 53-74(SIV); and Naldini et al. (1996), Science, 272: 263-267 (HIV) can be referred to.

In order to support incorporation of recombinant retrovirus into target cells, such recombinant retrovirus may comprise a viral polypeptide (e.g., retrovirus env). Such a viral polypeptide has been sufficiently established in the present technical field, and for instance, U.S. Patent No. 5,449,614 can be referred to. The viral polypeptide may be an amphotropic viral polypeptide, for example, amphotropic env. Such an amphotropic viral polypeptide supports incorporation of retrovirus into cells derived from a large number of species including cells that are out of the original host species. The viral polypeptide may be a xenotropic viral polypeptide supporting incorporation of retrovirus into cells that are out of the original host species. In another aspect of the present invention, the viral polypeptide is an ecotropic virus polypeptide, for example, ecotropic env, and such an ecotropic virus polypeptide supports incorporation of retrovirus into the cells of the original host species.

Examples of the viral polypeptide capable of supporting incorporation of retrovirus into cells include MMLV amphotropic env, MMLV ecotropic env, MMLV xenotropic env, vesicular stomatitis virus-g protein (VSV-g), HIV-1 env, gibbon ape leukemia virus (GALV) env, RD114, FeLV-C, FeLV-B, MLV10A1 env gene, and mutants thereof, such as chimera, but the examples are not limited thereto. For instance, Yee et al. (1994), Methods Cell Biol., PtA: 99-112(VSV-G); and U.S. Patent No. 5,449,614 can be referred to. In some cases, in order to promote expression or reinforced binding to a receptor, the viral polypeptide is genetically modified.

In general, recombinant virus is produced by introducing a viral DNA or RNA construct into producer cells. In some cases, the producer cells do not express an exogenous gene. In other cases, the producer cells are "packaging cells" comprising one or more exogenous genes, for example, genes encoding one or more of gag, pol, or env polypeptide, and/or one or more of retrovirus gag, pol, or env polypeptide. Retrovirus packaging cells may comprise a gene encoding a viral polypeptide, for example, VSV-g that supports incorporation of retrovirus into target cells. In some cases, the packaging cells comprise genes encoding one or more lentivirus proteins, such as gag, pol, env, vpr, vpu, vpx, vif, tat, rev, or nef. In some cases, the packaging cells comprise genes encoding adenovirus proteins, such as E1A or E1B, or other adenovirus proteins. For instance, proteins supplied by such packaging cells may be: retrovirus-derived proteins, such as gag, pol, and env; lentivirus-derived proteins, such as gag, pol, env, vpr, vpu, vpx, vif, tat, rev, and nef; and adenovirus-derived proteins, such as E1A and E1B. In many examples, the packaging cells supply proteins derived from viruses that are different from the virus as an origin of the viral vector.

Examples of the packaging cell line include cell lines that can be easily transfected, but the examples are not limited thereto. The packaging cell line can be based on 293T cells, NIH3T3, COS, or HeLa cell lines. The packaging cells are frequently used for packaging a viral vector plasmid comprising a deletion of at least one gene encoding a protein necessary for virus packaging. Cells capable of supplying a deleted protein or polypeptide from a protein encoded by such a viral vector plasmid may be used as packaging cells. Examples of the packaging cell line include Platinum-E (Plat-E); Platinum-A (Plat-A); BOSC23 (ATCCCRL11554); and Bing (ATCC CRL 1 1270), but are not limited thereto. For instance, Morita et al. (2000), Gene Therapy,7: 1063-1066; Onishi et al. (1996), Experimental Hematology, 24: 324-329; and U.S. Patent No. 6,995,009 can be referred to. Commercially available packaging lines are also useful, and examples of such a commercially available packaging line include Ampho-Pak293 cell line, Eco-Pak2-293 cell line, RetroPackPT67 cell line, and Retro-X Universal Packaging System (all of which are available from Clontech).

The retroviral construct may be derived from a certain range of retroviruses, such as MMLV, HIV-1, SIV or FIV, or from other retroviruses described in the present description. The retroviral construct may encode all viral polypeptides necessary for one or more replication cycles of a specific virus. In some cases, the efficiency of incorporation of virus is improved by addition of other factors or other viral polypeptides. In other cases, as described in U.S. Patent No. 6,872,528, a viral polypeptide encoded by a retroviral construct does not support more than one cycle of replication. Under such circumstances, promotion of virus incorporation can be supported by addition of other factors or other viral polypeptides. In the illustrative embodiment, the recombinant retrovirus is an HIV-1 virus, which comprises a VSV-g polypeptide but does not comprise an HIV-1 env polypeptide.

The retroviral construct may comprise a promoter, a multicloning site, and/or a resistance gene. Examples of the promoter include CMV, SV40, EF1α, β-actin; retrovirus LTR promoter, and an inducible promoter, but the examples are not limited thereto. The retroviral construct may also comprise a packaging signal (e.g., a packaging signal derived from an MFG vector; psi packaging signal). Examples of some retroviral constructs known in the present technical field include pMX, pBabeX, and derivatives thereof, but the examples are not limited thereto. For instance, Onishi et al. (1996), Experimental Hematology, 24: 324-329 can be referred to. In some cases, the retroviral construct is a self-inactivating lentiviral vector (SIN), and for instance, Miyoshi et al. (1998), J. Virol., 72(10): 8150-8157 can be referred to. In some cases, the retroviral construct is LL-CG, LS-CG, CL-CG, CS-CG, CLG, or MFG. Miyoshi et al. (1998), J. Virol., 72(10): 8150-8157; Onishi et al. (1996), Experimental Hematology, 24: 324-329; Riviere et al. (1995), PNAS, 92: 6733-6737 can be referred to. Examples of the viral vector plasmid (or construct) include: retrovirus-based vectors, such as pMXs, pMxs-IB, pMXs-puro, and pMXs-neo (wherein pMXs-IB is a vector that supports a blasticidin resistance gene, instead of a puromycin resistance gene supported by pMXs-puro; Kimatura et al. (2003), Experimental Hematology, 31: 1007-1014; MFG Riviere et al. (1995), Proc. Natl. Acad. Sci. U.S.A., 92: 6733-6737; pBabePuro; Morgenstern et al. (1990), Nucleic Acids Research, 18: 3587-3596; LL-CG, CL-CG, CS-CG, CLG Miyoshi et al. (1998), Journal of Virology, 72: 8150-8157, etc.); and adenovirus-based vectors, such as pAdex1 (Kanegae et al. (1995), Nucleic Acids Research, 23: 3816-3821, etc.). In the illustrative embodiment, the retroviral construct comprises blasticidin (e.g., pMXs-IB), puromycin (e.g., pMXs-puro and pBabePuro); or neomycin (e.g., pMXs-neo). For instance, Morgenstern et al. (1990), Nucleic Acids Research, 8: 3587-3596 can be referred to.

Methods for producing recombinant virus from packaging cells and the use thereof have been sufficiently established; and for instance, U.S. Patent Nos. 5,834,256; 6,910,434; 5,591,624; 5,817,491; 7,070,994; and 6,995,009 can be referred to. A majority of methods start with introduction of a viral construct into a packaging cell line. Introduction of such a viral construct includes a calcium phosphate method, a lipofection method (Felgner et al. (1987) Proc. Natl. Acad. Sci. U.S.A. 84: 7413-7417), an electroporation method, a microinjection method, FuGENE Transfection, etc., and any method described in the present description, but is not limited thereto. The viral construct may be introduced into host fibroblasts by any method known in the present technical field.

A nucleic acid construct may be introduced into host cells by applying various well-known methods, such as non-viral transfection of cells. In the illustrative aspect, the construct is incorporated into a vector, and is then introduced into host cells. Introduction of the construct into cells includes electroporation, calcium phosphate-mediated transition, nucleofection, sonoporation, heat shock, magnetofection, liposome-mediated transition, microinjection, microprojectile-mediated transition (nanoparticles), cationic polymer-mediated transition (DEAE dextran, polyethyleneimine, polyethylene glycol (PEG), etc.), and cell fusion, but is not limited thereto. Introduction of the construct into cells may be carried out by any non-viral transfection known in the present technical field. Other examples of transfection include transfection reagents, such as Lipofectamine, Dojindo Hilymax, Fugene, jetPEI, Effectene, and DreamFect.

### [Fibroblasts comprising exogenous gene(s)]

The present invention includes fibroblasts comprising an exogenous Tbx6 gene. In addition, the present invention includes fibroblasts comprising an exogenous Tbx6 gene, an exogenous SRF gene, and an exogenous Myocd gene. In another aspect of the present invention, the fibroblasts comprising an exogenous gene(s) of the present invention are in an *in vitro* state. In another aspect of the present invention, the fibroblasts comprising an exogenous gene(s) of the present invention are mammalian cells, such as human cells, or are induced from human cells.

### [Fibroblast-derived cardiac progenitor cells or fibroblast-derived cardiomyocytes comprising exogenous gene(s)]

The present invention further relates to fibroblast-derived cardiac progenitor cells (induced cardiac progenitor cells, cardiac progenitor cell-like cells) or fibroblast-derived cardiomyocytes (induced cardiomyocytes, cardiomyocyte-like cells), which are produced by the above-described method for producing cardiac progenitor cells or cardiomyocytes. In the present invention, "fibroblast-derived" cardiac progenitor cells or cardiomyocytes mean cardiac progenitor cells or cardiomyocytes, which are induced from fibroblasts. Since the induced cardiac progenitor cells or induced cardiomyocytes of the present invention are induced from fibroblasts comprising an exogenous gene(s), the induced cardiac progenitor cells or induced cardiomyocytes of the present invention also comprise an exogenous Tbx6 gene, or an exogenous Tbx6 gene, an exogenous SRF gene and an exogenous Myocd gene. In another aspect of the present invention, the induced cardiac progenitor cells or induced cardiomyocytes of the present invention are in *an in vitro* state. In another aspect of the present invention, the induced cardiac progenitor cells or induced cardiomyocytes of the present invention are mammalian cells such as human cells, or are derived from mammalian cells such as human cells.

As described above, whether or not the cells induced from fibroblasts are cardiac progenitor cells can be confirmed using the expression of a marker specific to cardiac progenitor cells. The cells, in which the expression of a cardiac progenitor cell-specific marker has been confirmed, are also referred to as "cardiac progenitor cell-like cells."

Likewise, as described above, whether or not the cells induced from fibroblasts are cardiomyocytes can be confirmed using the expression of a marker specific to cardiomyocytes. The cells, in which the expression of a cardiac progenitor cell-specific marker has been confirmed, are also referred to as "cardiomyocyte-like cells."

### Marker expression can be confirmed at a gene level or at a protein level.

After the induced cardiac progenitor cells of the present invention have been induced from fibroblasts, the cells can be maintained as cardiac progenitor cells for a certain period of time or longer. That is to say, the cardiac progenitor cells induced by the method of the present invention can be characterized in that, after introduction of a Tbx6 gene into the fibroblasts, the induced cardiac progenitor cells express cardiac progenitor cell-specific markers, such as T, Mesp1, or Flk1 (KDR) for a certain period of time (e.g., for 3 weeks, 4 weeks, or 5 weeks) or longer.

The present invention also provides a composition comprising fibroblasts comprising an exogenous gene(s), or fibroblast-derived cardiac progenitor cells or fibroblast-derived cardiomyocytes comprising an exogenous gene(s). The composition of the present invention comprises the above-described fibroblasts, or induced cardiac progenitor cells or induced cardiomyocytes, and may further comprise, as suitable components, salts; a buffer; a stabilizer; a protease inhibitor; a cell membrane and/or cell wall preserving compound, such as glycerol or dimethyl sulfoxide; a nutrient medium suitable for cells; and the like.

### [Inducer]

The present invention also provides an inducer for inducing cardiac progenitor cells from fibroblasts, or an inducer for inducing cardiomyocytes from fibroblasts. Moreover, since smooth muscle cells or endothelial cells are also induced by introducing a Tbx6 gene, an SRF gene and a Myocd gene into fibroblasts, the present invention also provides an inducer for inducing smooth muscle cells from fibroblasts, or an inducer for inducing vascular endothelial cells from fibroblasts.

In another aspect of the present invention, the inducer for inducing cardiac progenitor cells of the present invention comprises, at least, either 1) a Tbx6 polypeptide or 2) a nucleic acid comprising a nucleotide sequence encoding the Tbx6 polypeptide. In another aspect of the present invention, the inducer for inducing cardiomyocytes, inducer for inducing smooth muscle cells, or inducer for inducing vascular endothelial cells of the present invention comprises, at least, either 1) a mixture of a Tbx6 polypeptide, an SRF polypeptide and a Myocd polypeptide, or 2) a mixture of a nucleic acid comprising a nucleotide sequence encoding a Tbx6 polypeptide, a nucleic acid comprising a nucleotide sequence encoding an SRF polypeptide, and a nucleic acid comprising a nucleotide sequence encoding a Myocd polypeptide.

The inducer of the present invention may comprise one or more selected from: salts such as NaCl, MgCl, KCl, or MgSO₄; buffers such as a Tris buffer, N-(2-hydroxyethyl)piperazine-N'-2-ethanesulfonic acid (HEPES), 2-(N-morpholino)ethanesulfonic acid (MES), 2-(N-morpholino)ethanesulfonic acid sodium salt (MES), 3-(N-morpholino)propanesulfonic acid (MOPS), or N-tris[hydroxymethyl]methyl-3-aminopropanesulfonic acid (TAPS); solubilizers; detergents including nonionic detergents such as Tween-20; protease inhibitors; glycerol; etc., in addition to the above-described polypeptides or nucleic acids. Moreover, the inducer of the present invention may comprise a reagent for introducing the polypeptides or nucleic acids of reprogramming factors into fibroblasts.

The inducer of the present invention may be directly administered to an individual body (e.g., into cardiac tissues). The inducer of the present invention is useful for inducing fibroblasts to cardiac progenitor cells or cardiomyocytes, smooth muscle cells, or vascular endothelial cells, and this induction can be carried out *in vitro* or *in vivo.* Induction of fibroblasts to cardiac progenitor cells or cardiomyocytes can be used to treat various heart failures.

Accordingly, the inducer of the present invention may comprise a pharmaceutically acceptable excipient. Examples of a suitable excipient include water, saline, dextrose, glycerol, ethanol, and a combination thereof. Moreover, as desired, the present inducer may comprise a small amount of auxiliary substance, such as a wettable powder, an emulsifier, or a buffer. Actual methods for preparing such dosage forms have been known. For instance, Remington's Pharmaceutical Sciences, Mack Publishing Company, Easton, Pennsylvania, 17th edition, 1985 can be referred to.

A pharmaceutically acceptable excipient, such as a vehicle, an adjuvant, a carrier or a diluent, can be easily obtained. Further, a pharmaceutically acceptable auxiliary substance, such as a pH adjuster, a buffer, a tension adjuster, a stabilizer or a wettable powder, can be easily purchased.

### [Therapeutic method using cells]

The fibroblasts comprising an exogenous gene(s) of the present invention can be used to treat an individual in need of the treatment. Likewise, the induced cardiac progenitor cells or induced cardiomyocytes of the present invention can be used to treat an individual in need of the treatment. The fibroblasts comprising an exogenous gene(s) of the present invention, or the induced cardiac progenitor cells or induced cardiomyocytes of the present invention, can be introduced into a recipient individual (an individual in need of treatment), and in such a case, introduction of the fibroblasts comprising an exogenous gene(s) of the present invention, or the induced cardiac progenitor cells or induced cardiomyocytes of the present invention, into such a recipient individual provides the treatment of the condition or disorder of the individual. Therefore, the present invention relates to a therapeutic method comprising administering the fibroblasts comprising an exogenous gene(s) of the present invention, or the induced cardiac progenitor cells or induced cardiomyocytes of the present invention, to an individual.

For example, in some embodiments, the therapeutic method of the present invention comprises: i) generating induced cardiac progenitor cells or induced cardiomyocytes *in vitro*; and ii) introducing the induced cardiac progenitor cells or the induced cardiomyocytes into an individual in need thereof.

In addition, the present invention provides a method for reprogramming fibroblasts *in vivo* in cardiac tissues. The present method can be utilized to treat an individual. In some embodiments, the therapeutic method of the present invention comprises allowing an inducer or a composition containing a reprogramming factor(s) to come into contact with the fibroblasts of an individual *in vivo.* The contact comprises administration of the inducer or reprogramming composition of the present invention into a therapeutic site or a site close thereto, for example, into the heart or in the periphery thereof, in an individual body. The administration method is, for example, a method comprising inducing a catheter that has been inserted into the end of the artery to a site close to the diseased cardiac portion, and then injecting the inducer or composition comprising a reprogramming factor(s) of the present invention into the fibroid diseased tissues, through the tip of the catheter.

The therapeutic method of the present invention is useful to treat an individual suffering from cardiac or cardiovascular diseases or disorders, such as cardiovascular disease, aneurysm, angina, arrhythmia, atherosclerosis, cerebrovascular accidental disease (stroke), cardiovascular disease, congenital heart disease, congestive heart failure, myocarditis, coronary venous valve disease, scalability artery disease, diastolic dysfunction, endocarditis, hypertension, cardiomyopathy, hypertrophic cardiomyopathy, restrictive cardiomyopathy, coronary disease resulting in ischemic cardiomyopathy, mitral valve prolapse, myocardial infarction (heart attack), or venous thromboembolism.

The unit dosage form of an induced cardiomyocyte population, or a population of fibroblasts, induced cardiac progenitor cells or induced cardiomyocytes, may comprise approximately 10³ to approximately 10⁹ cells, for example, approximately 10³ to approximately 10⁴, approximately 10⁴ to approximately 10⁵, approximately 10⁵ to approximately 10⁶, approximately 10⁶ to approximately 10⁷, approximately 10⁷ to approximately 10⁸, or approximately 10⁸ to approximately 10⁹ cells.

### Examples

### [Example 1] Production of mouse embryonic fibroblasts (MEF)

### (1) Production of mouse embryonic fibroblasts (MEF)

Female ICR mice (CLEA Japan, Inc.) at 7 to 10 weeks after birth were mated with Mesp1-GFP transgenic mice (male) (Development 126, 3437-3447 (1999), "MesP1 is expressed in the heart precursor cells and required for the formation of a single heart tube"). The day on which fertilization was confirmed was defined as Day 0 of pregnancy, and on Day 12 after confirmation of the pregnancy, embryos were excised from the pregnant ICR mice. Then, the heart was excised from each embryo, and the excised heart was then imaged with fluorescence under an inverted microscope (IX71, Olympus). Thereafter, embryos emitting GFP fluorescence were selected.

From the selected embryos, four limbs, and solid organs such as 1/2 to 2/3 of head portion, lung, liver, kidney and intestinal tract were excised. The tissues of the remaining trunk of the body were washed with PBS (phosphate buffered saline)(-) (045-29795, WAKO), so that the blood cell components were fully removed. Thereafter, using sterilized surgical scissors, the tissues were sheared into as many as sections. To the sheared tissue sections, a solution comprising 0.25 %Trypsin-EDTA (25200-072, Gibco) and PBS (-) (045-29795, WAKO) at a mixing ratio of I : 1 was added (15 mL/6 to 7 embryos), and the cells were then incubated while shaking at 37°C for 15 minutes in a water bath. After that, 15 mL of a stock solution of FBS (Fetal Bovine Serum) (SV30014.03, Thermo Scientific) was added to the mixture, and was then fully suspended therein. The obtained suspension was centrifuged at 1500rpm for 5 minutes at 4°C, and a supernatant was then removed.

The cell precipitate and suspension were re-suspended in 30 mL of a medium for MEF (10% FBS/DMEM/PSA) (Table 1), and the obtained re-suspension was inoculated in a 10-cm dish for tissue culture (172958, Thermo Scientific), so that the re-suspension corresponding to 2 or 3 embryos could be inoculated in the single 10-cm dish. The cells were cultured under conditions of 37°C/5% CO₂, and on the following day, the medium was exchanged with a fresh medium for MEF. Thereafter, medium exchange was continuously carried out every 3 or 4 days.

### [Table 1]

**(Table 1) Medium for MEF**

| | |
|---|---|
| FBS (Fetal Bovine Serum) (Thermo Scientific, SV30014.03) | 50 mL |
| DMEM (WAKO, 044-29765) | 440 mL |
| PSA | 5 mL |
| Sodium Pyruvate (Sigma, S8636) | 5 mL |
| GlutaMAX (Gibco, 35050-061) | 5 mL |
| Non-essential amino acids solution 100x (Sigma, M7145) | 5 mL |

### (2) Sorting of mouse embryonic fibroblasts (MEF) by flow cytometry

When mouse embryonic fibroblasts (MEF) were induced to differentiate into cardiac progenitor cells, sorting was carried out by flow cytometry (FACS) as follows, and GFP (-) cells were used.

The medium was aspirated, and the cells were then washed with PBS (-). Thereafter, 2 mL of 0.25% Trypsin-EDTA was added to each dish, and the cells were then left at rest under conditions of 37°C/5% CO₂ for 5 minutes. After the floating of the cells in the culture medium had been confirmed, the reaction was neutralized with 8 mL of a medium for MEF (10% FBS/DMEM/PSA) (Table 1), and the cells were then recovered in a 15-mL tube (430791, Corning). The recovered cells were centrifuged under conditions of 1500 rpm/5 minutes/4°C. After aspiration of the supernatant, 350 µL of a solution for performing FACS (5% FBS/PBS) (Table 2) was added to the cells, and the cells were then fully suspended. This suspension was filtered using a 5-mL polystyrene round tube with cell strainer cap (REF 353335, FALCON), so as to obtain a sample for use in FACS. Using FACS (FACS AriaIII, Nippon Becton Dickinson Company, Ltd.), GFP-positive cells were separated from GFP-negative cells in the above-described sample, and the negative cells were used to induce differentiation into cardiac progenitor cells.

### [Table 2]

**(Table 2) Solution for performing FACS**

| | |
|---|---|
| FBS (Fetal Bovine Serum) (Thermo Scientific, SV30014.03) | 10 mL |
| PBS (-) (WAKO, 045-29795) | 190 mL |

### [Example 2] Production of induced cardiac progenitor cells and cell culture

Plat-E packaging cells were inoculated at a concentration of 3.6 x 10⁶ cells in a gelatin-coated 10-cm dish for tissue culture (172958, Thermo Scientific), and were then left at rest under conditions of 37°C/5% CO₂ (Day 1).

On the following day (Day 2), 27 µL of FuGENE 6 Transfection Reagent (E2691, Promega) was mixed into 300 µL of Opti-MEM (31985-070, Gibco). After the mixed solution had been left at rest for 5 minutes, 9000 ng of a retrovirus plasmid of pMx-Tbx6 (see Cell 142, 375-386, August 6, 2010, "Direct Reprogramming of Fibroblasts into Functional Cardiomyocytes by Defined Factors") was added to the mixed solution, followed by strong tapping, and thereafter, the obtained mixture was left at rest at room temperature for 15 minutes. The thus obtained solution was added dropwise to the Plat-E cells as a whole that had been prepared on the previous day (Day 1), and the dish was then left at rest under conditions of 37°C/5% CO₂ (transfection).

Twenty-four hours later (Day 3), the medium was exchanged with a Plat-E culture medium (DMEM/10%FBS/PSA) (Table 3), and it was then left at rest for further 24 hours under conditions of 37°C/5% CO₂.

### [Table 3]

**(Table 3) Medium for Plat-E**

| | |
|---|---|
| FBS (Fetal Bovine Serum) (Thermo Scientific, SV30014.03) | 50 m L |
| DMEM (WAKO, 044-29765) | 440 mL |
| PSA | 5 m L |
| Sodium Pyruvate (Sigma, S8636) | 5 mL |
| GlutaMAX (Gibco, 35050-061) | 5 mL |
| Non-essential amino acids solution 100x (Sigma, M7145) | 5 mL |

Meanwhile, the MEF of Example 1 was inoculated at a concentration of 0.5 x 10⁵ cells/well in a 12 Well Cell Culture Multiwell Plate (353043, FALCON), and was then cultured using a medium for MEF under conditions of 37°C/5% CO₂.

Forty-eight hours later (Day 4), each culture supernatant was filtrated through a Minisart filter with a pore size of 0.45 µm (17598, Sartorius Stedim Biotech), and was then recovered in a 50-mL tube (430829, Corning). Into 10 mL of the recovered supernatant, 4 µL of Polybrene Transfection Reagent (10 mg/mL) (#TR-1003-G, Millipore) was mixed. The obtained solution was defined as a retrovirus solution used for the Tbx6 gene.

The medium of the MEF of Example 1, which had been inoculated in the 12 Well Cell Culture Multiwell Plate on the previous day (Day 3), was aspirated, and the medium was then exchanged with a Tbx6 retrovirus solution, so that the cells were infected with the virus (infection).

On the following day of the gene introduction, the medium was exchanged with a medium for reprogramming of cardiac progenitor cells (Table 4), and the cells were then cultured under conditions of 37°C/5% CO₂. Thereafter, the medium was exchanged with a fresh medium every 3 or 4 days, while the cell culture was continued.

### [Table 4]

**(Table 4) Medium for reprogramming of cardiac progenitor cells**

| | |
|---|---|
| FBS (Fetal Bovine Serum) (Thermo Scientific, SV30014.03) | 50 mL |
| DMEM (WAKO, 044-29765) | 440 mL |
| PSA | 5 mL |

### [Example 3] Production of induced cardiomyocytes using Tbx6, SRF and Myocd, and cell culture

### (1) Method of introducing Tbx6, SRF and Myocd genes, using retrovirus

A virus solution was produced in the same manner as "Production of induced cardiac progenitor cells" of Example 2. In the present example, however, three genes were introduced into the cells. On Day 1, Plat-E cells were prepared in three 10-cm dishes for tissue culture by the same method as that applied in Example 2. On Day 2, 27 µL of FuGENE 6 Transfection Reagent (E2691, Promega) was mixed into 300 µL Opti-MEM (31985-070, Gibco). After the mixed solution had been left at rest for 5 minutes, 9000 ng of a retrovirus plasmid of each of pMx-Tbx6, pMx-SRF, and pMx-Myocd (see Cell 142, 375-386, August 6, 2010, "Direct Reprogramming of Fibroblasts into Functional Cardiomyocytes by Defined Factors" with respect to their production technique) was added to the mixed solution, followed by strong tapping, and thereafter, the obtained mixture was left at rest at room temperature for 15 minutes. The thus obtained solution was added dropwise to the Plat-E cells as a whole that had been prepared on the previous day (Day 1), and the dishes were then left at rest under conditions of 37°C/5% CO₂ (transfection).

Twenty-four hours later (Day 3), the medium was exchanged with a Plat-E culture medium (DMEM/10%FBS/PSA) (Table 3), and it was then left at rest for further 24 hours under conditions of 37°C/5% CO₂. Forty-eight hours later (Day 4), each culture supernatant was filtrated through a Minisart filter with a pore size of 0.45 µm (17598, Sartorius Stedim Biotech), and was then recovered in a 50-mL tube (430829, Corning). Into 10 mL of the recovered supernatant, 4 µL of Polybrene Transfection Reagent (10 mg/mL) (#TR-1003-G, Millipore) was mixed. The obtained solution was defined as a retrovirus solution used for each gene (Tbx6, SRF, and Myocd).

On Day3, a Matrigel solution (70.4 µg/mL) prepared by diluting 0.25 mL of Matrigel Growth Factor Reduced (354230, Corning) with 28.2 mL of DMEM was added in an amount of 0.25 mL to each well of a 12 Well Cell Culture Multiwell Plate (353043, FALCON), and it was then left at rest for 1 hour at 37°C, so that the plate was coated with Matrigel. After the removal of the solution, MEF (Example 1) was inoculated therein at a concentration of 0.5 x 10⁵ cells/well. Thereafter, the culture was continued using a medium for MEF (Table 1).

On Day4, the medium for MEF was aspirated from the plate prepared on Day 3, and the medium was then exchanged with a retrovirus solution comprising Tbx6, SRF, and Myocd in equal amounts, so that the MEF was infected with the virus (infection).

### (2) Cell culture using cardiomyocyte induction medium

On the day following the gene introduction (Day 5), the medium was exchanged with an FFV medium (Table 5), and the culture was then carried out under conditions of 37°C/5% CO₂. Thereafter, the medium was exchanged with a fresh medium every 3 or 4 days, while the cell culture was carried out.

### [Table 5]

**(Table 5) FFV Medium**

| | |
|---|---|
| StemPro(R) 34 SFM (Gibco, 10639-011) with Nutrient Supplement | 10 mL |
| GlutaMAX (Gibco, 35050-061) | 100 µL |
| 5 mg/ mL Ascrobic Acid (Sigma, A-4544) | 200 µL |
| 5 ng/ µL Recombinant Human VEGF165 (R & D Systems, 293-VE-050) | 10 µL |
| 10 ng/ µL Recombinant Human FGF basic 146 aa (R & D Systems, 233-FB-025) | 10 µL |
| 50 ng/ µL Recombinant Human FGF10 (R & D Systems, 345-FG-025) | 5 µL |

### [Example 4] Induction of cardiac progenitor cells from mouse fibroblasts

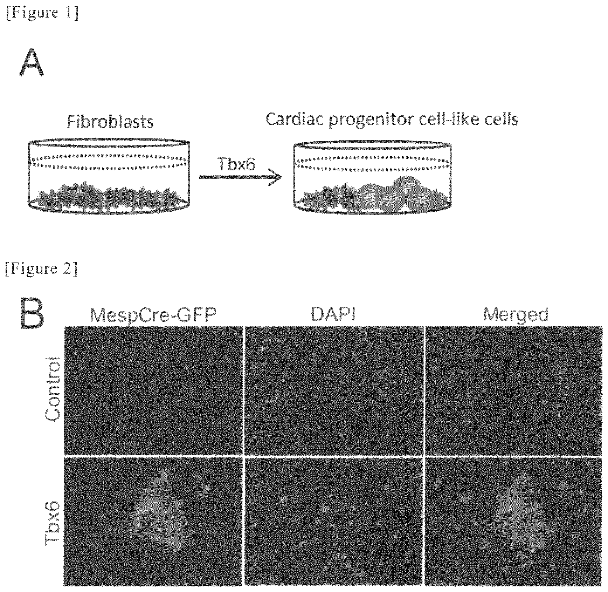

A Tbx6 gene was introduced into mouse fibroblasts (MEF), so that the MEF was induced to differentiate into cardiac progenitor cells (Figure 1, Example 2).

Mesp1-Cre GFP flox mouse fibroblasts express GFP when expressing a cardiac progenitor cell-specific transcriptional factor Mesp1. A Tbx6 gene was introduced into the Mesp1-Cre GFP flox mouse fibroblasts (Example 2), and thereafter, it was confirmed that Mesp1-expressing GFP-positive cells formed colonies (Figure 2).

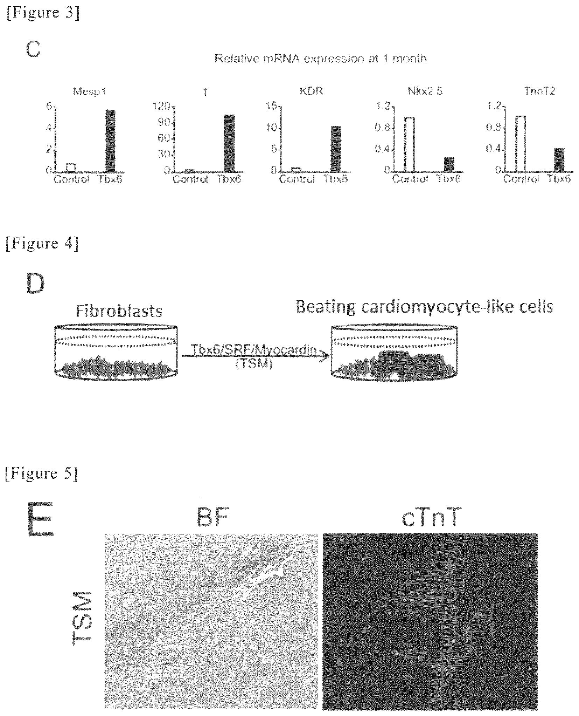

Subsequently, the mRNA expression of a differentiation marker gene was measured in the cells, into which the Tbx6 gene had been introduced. One month after the induction, the expression of cardiac progenitor cell-specific genes Mesp1 and T (brachyury), and further, the expression of KDR were maintained. On the other hand, the expression of Nkx2.5 or troponinT, which is expressed in differentiated cardiomyocytes, was not found (Figure 3).

Examples 2 and 4 demonstrated that fibroblasts are induced to differentiate into cardiac progenitor cells by introducing Tbx6 into the fibroblasts.

### [Example 5] Induction of cardiomyocytes from mouse fibroblasts

The Tbx6, SRF and Myocd genes were introduced into mouse fibroblasts (MEF), so that the MEF was induced to differentiate into cardiomyocytes (Figure 4, Example 3).

The forms of the induced cardiomyocytes were changed, and the induced cardiomyocytes exhibited a striated structure (Figure 5). In addition, the expression of troponinT (cTnT) as a structural protein of the cardiac muscle, or the expression of SM-MHC as a protein of smooth muscle cells, was found (Figure 5).

The mRNA expression of the induced cells as a whole was examined. As a result, it was demonstrated that cardiomyocyte-specific genes that had not been induced by the single use of Tbx6, such as Nkx2.5 or troponinT (Tnnt2), were expressed, in addition to the induction of cardiac progenitor cell genes (Mesp1, T, and KDR).

Examples 3 and 5 demonstrated that fibroblasts are induced to differentiate into cardiomyocytes by introducing the Tbx6, SRF and Myocd genes into the fibroblasts.

### [Example 6] Induction of smooth muscle cells and vascular endothelial cells from mouse fibroblasts

The Tbx6, SRF, and Myocd genes were introduced into mouse fibroblasts (MEF) by the same method as that applied in Example 3.

On Day 14 after the gene introduction, immunostaining was carried out using an anti-Smooth muscle myosin heavy chain (myosin-11 or SMMHC) antibody.

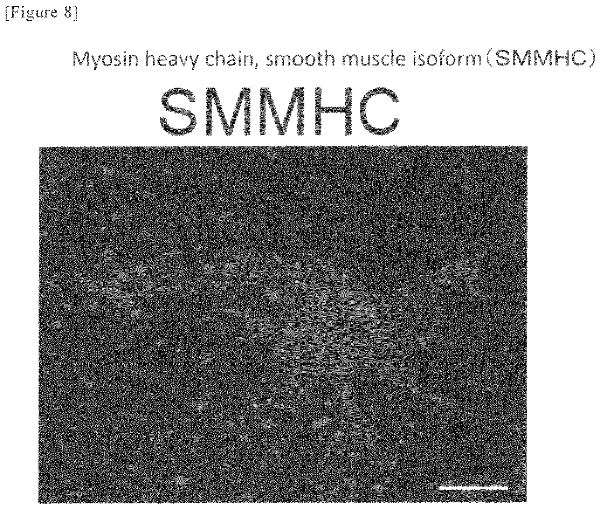

As a result, the cells, into which the Tbx6, SRF and Myocd genes had been introduced, exhibited positive to the aforementioned antibody. Thus, it is found that the cells expressed a myosin heavy chain that is a feature of the smooth muscle (Figure 8).

Moreover, on Day 14 after the gene introduction, the expression levels of myosin heavy polypeptide 11 (Myh11) and Platelet/endothelial cell adhesion molecule 1 (Pecam1) were measured according to qRT-PCR. Myh11 is a protein specifically expressed in the smooth muscle, whereas Pecam1 is a protein specifically expressed in vascular endothelial cells.

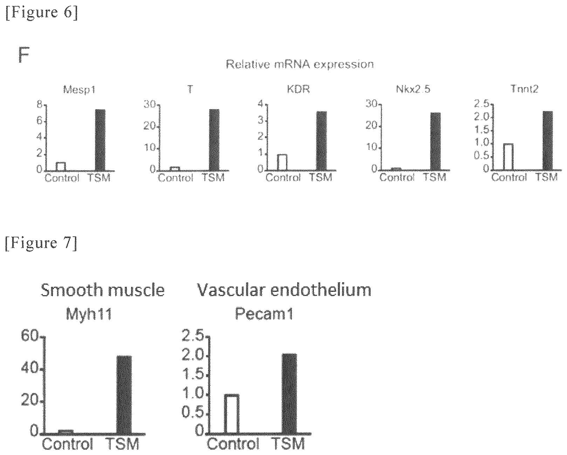

As a result, it was confirmed that the expression of Myh11 and Pecaml was induced in the cells into which the Tbx6, SRF and Myocd genes had been introduced (TSM) (Figure 7).

The present example demonstrated that smooth muscle cells or vascular endothelial cells can be induced by introducing the Tbx6, SRF and Myocd genes into fibroblasts (MEF).

### Industrial Applicability

According to the present invention, a method for directly producing cardiac progenitor cells from fibroblasts and a method for directly producing cardiomyocytes from fibroblasts are provided. In addition, the present invention can provide cardiac progenitor cells and cardiomyocytes, which are produced by the method of the present invention. Since the cardiac progenitor cells induced from fibroblasts according to the present invention have maintained the expression of a plurality of cardiac progenitor cell genes, the present invention can provide a method for producing cardiac progenitor cells, which is more stable than conventional methods, and cardiac progenitor cells. Since cardiac progenitor cells have proliferation ability, the cardiac progenitor cells produced by the present invention can be preferably applied to medical use.

Moreover, the cardiomyocytes induced by the present invention have been confirmed to beat, and further, the expression of a cardiac muscle-specific gene or the expression of a structural protein has been confirmed in the present cardiomyocytes. Therefore, the method for producing cardiac muscle of the present invention can provide functionally mature cardiomyocytes.

### Sequence Listing Free Text

SEQ ID NO: 1: Nucleotide sequence of human Tbx6.
SEQ ID NO: 2: Amino acid sequence of human Tbx6.
SEQ ID NO: 3: Nucleotide sequence of mouse Tbx6.
SEQ ID NO: 4: Amino acid sequence of mouse Tbx6.
SEQ ID NO: 5: Nucleotide sequence of human SRF.
SEQ ID NO: 6: Amino acid sequence of human SRF.
SEQ ID NO: 7: Nucleotide sequence of mouse SRF.
SEQ ID NO: 8: Amino acid sequence of mouse SRF.
SEQ ID NO: 9: Nucleotide sequence of human Myocd.
SEQ ID NO: 10: Amino acid sequence of human Myocd.
SEQ ID NO: 11: Nucleotide sequence of mouse Myocd.
SEQ ID NO: 12: Amino acid sequence of mouse Myocd.

## Claims

1. A method for producing cardiac progenitor cells, comprising a step of introducing a Tbx6 gene into fibroblasts.

2. A method for producing cardiomyocytes, comprising a step of introducing a Tbx6 gene, an SRF gene and a Myocardin gene into fibroblasts.

3. A cardiac progenitor cell derived from a fibroblast, comprising an exogenous Tbx6 gene.

4. A cardiomyocyte derived from a fibroblast, comprising an exogenous Tbx6 gene, an exogenous SRF gene and an exogenous Myocardin gene.

5. An inducer for inducing cardiac progenitor cells from fibroblasts, wherein the inducer comprises a Tbx6 gene.

6. An inducer for inducing cardiomyocytes from fibroblasts, wherein the inducer comprises a Tbx6 gene, an SRF gene and a Myocd gene.

7. An inducer for inducing smooth muscle cells from fibroblasts, wherein the inducer comprises a Tbx6 gene, an SRF gene and a Myocd gene.

8. An inducer for inducing vascular endothelial cells from fibroblasts, wherein the inducer comprises a Tbx6 gene, an SRF gene and a Myocd gene.
